# EUROPEAN PATENT APPLICATION

(11) **EP 4 155 389 A2**
(43) Date of publication of application: **29.03.2023**
(21) Application number: 22207135.9
(22) Date of filing: 26.03.2021
(51) Int. Cl.: C12N 5/0775, A61K 35/28

(54) **METHOD OF PRODUCING PROLIFERATED CELLS, METHOD OF PRODUCING CELL PRODUCT, MESENCHYMAL STEM CELL POPULATION AND METHOD OF PRODUCING SAME, CULTURE SUPERNATANT OF STEM CELLS AND METHOD OF PRODUCING SAME, AND THERAPEUTIC AGENT**

(30) Priority: 31.03.2020 JP 2020063408
(62) Divisional of application: 21779739.8
(71) Applicant: Cell Exosome Therapeutics Inc., Tokyo 150-0011 (JP)
(72) Inventor: YANAGITA, Yasutomo, 150-0011 Tokyo (JP); RINOIE, Chugo, 150-0011 Tokyo (JP); ISHIDAO, Takefumi, 150-0011 Tokyo (JP); MINAMI, Itsunari, 150-0011 Tokyo (JP)
(74) Representative: AWA Sweden AB

(57) **Abstract**

A method of producing proliferated cells, the method including culturing cells, which have been seeded at a cell density of 0.002 to 2000 cells/cm², through adhesion culture in a proliferation culture medium in the presence of a culture substrate selected from a laminin fragment having integrin-binding activity and a modified form thereof, thereby proliferating the cells.

## Description

### FIELD

The present invention relates to a method of producing proliferated cells, a method of producing a cell product, a mesenchymal stem cell population and a method of producing the same, a culture supernatant of stem cells and a method of producing the same, and a therapeutic agent.

### BACKGROUND

Stem cells such as mesenchymal stem cells have garnered worldwide attention for their application to regenerative medicine. In order to apply stem cells to regenerative medicine, it is necessary to develop a culture technique for stable cell culturing and proliferation.

In order to culture, proliferate and mass-produce stem cells, it is common to first seed stem cells in a culture vessel at a certain cell density, and culture the cells until 80 to 90% or more of the culture vessel surface area is covered with the cells (a so-called "confluent state"), then peel off the cells using an enzyme such as trypsin and subculture the obtained cells in multiple new culture vessels (see, for example, Patent Literature 1).

On the other hand, stem cells such as mesenchymal stem cells are known to secrete cell-derived components such as various cytokines and exosomes, and are expected to be applied to medical treatment.

### CITATION LIST

### PATENT LITERATURE

Patent Literature 1: International Publication No. 2014/035215

### SUMMARY

### TECHNICAL PROBLEM

When mass-producing proliferated cells or cell-derived components using a cell culture technique, it is necessary to efficiently proliferate cells or to efficiently collect a culture supernatant containing cell-derived components.

Accordingly, an object of the present invention is to provide a cell culture technique with which cells seeded at a low cell density can be proliferated at a high proliferation ratio. Another object of the present invention is to provide a cell culture technique which has developed further from the aforementioned cell culture technique and can produce large amounts of cell products via a simple method while maintaining the adhered state of the obtained proliferated cells.

Still another object of the present invention is to provide a mesenchymal stem cell population having novel characteristics based on the above-described cell culture techniques, and to provide a culture supernatant of stem cells, containing large amounts of cell products such as cytokines based on the above-described cell culture techniques. A further object of the present invention is to provide a therapeutic agent containing the mesenchymal stem cell population or the culture supernatant described above.

### SOLUTION TO PROBLEM

According to an aspect, there is provided a method of producing proliferated cells, the method including: culturing cells, which have been seeded at a cell density of 0.002 to 2000 cells/cm², through adhesion culture in a proliferation culture medium in a presence of a culture substrate selected from a laminin fragment having integrin-binding activity and a modified form thereof, thereby proliferating the cells.

According to another aspect, there is provided a method of producing a cell product, the method including:
culturing cells in a proliferation culture medium according to the "method of producing proliferated cells" described above, thereby obtaining proliferated cells in an adhered state; and
culturing the proliferated cells in a production culture medium while maintaining the adhered state, thereby causing the cells to produce a cell product.

According to still another aspect, there is provided a method of producing a cell product, the method including:
culturing cells in a proliferation culture medium according to the "method of producing proliferated cells" described above, thereby obtaining proliferated cells in an adhered state; and
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells.

According to still another aspect, there is provided a method of producing a mesenchymal stem cell population having a reduced HLA-ABC positive rate, the method including: culturing mesenchymal stem cells in a proliferation culture medium in a presence of a culture substrate selected from a laminin fragment having integrin-binding activity and a modified form thereof, thereby obtaining proliferated cells.

According to still another aspect, there is provided a mesenchymal stem cell population, including HLA-ABC positive mesenchymal stem cells at a ratio of 70% or less.

According to still another aspect, there is provided a method of producing a culture supernatant of stem cells, the method including:
culturing stem cells through adhesion culture in a proliferation culture medium in a presence of a culture substrate selected from a laminin fragment having integrin-binding activity and a modified form thereof, thereby obtaining proliferated cells in an adhered state;
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells, and the method further includes collecting a supernatant of the production culture medium after performing the culturing in the production culture medium.

According to still another aspect, there is provided a culture supernatant of stem cells, the culture supernatant containing 5000 pg/mL or more of HGF.

According to still another aspect, there is provided a culture supernatant of stem cells, the culture supernatant containing 50 pg/mL or more of CD9/CD63 EC domain fusion protein.

According to still another aspect, there is provided a therapeutic agent containing the mesenchymal stem cell population or the culture supernatant described above.

### ADVANTAGEOUS EFFECTS OF INVENTION

According to the present invention, it is possible to provide a cell culture technique with which cells seeded at a low cell density can be proliferated at a high proliferation ratio. According to the present invention, it is also possible to provide a cell culture technique further developed from the aforementioned cell culture technique which can produce large amounts of cell products via a simple method while maintaining the adhered state of the obtained proliferated cells.

According to the present invention, it is possible to provide, based on the above-described cell culture techniques, a mesenchymal stem cell population having novel characteristics and to provide a culture supernatant of stem cells, containing large amounts of cell products such as cytokines. According to the present invention, it is also possible to provide a therapeutic agent containing the mesenchymal stem cell population or the culture supernatant described above.

### BRIEF DESCRIPTION OF THE DRAWINGS

FIG. 1 is a graph showing the number of proliferated cells (comparative example).
FIG. 2 is a microscopic image of cells obtained after 20 days of culturing at a seeding density of 10 cells/cm² (comparative example).
FIG. 3 is a graph showing the number of proliferated cells (example of the present invention).
FIG. 4 is a microscopic image of cells obtained after 20 days of culturing at a seeding density of 10 cells/cm² (example of the present invention).
FIG. 5 is a graph showing the number of proliferated cells (referential example).
FIG. 6 is a microscopic image of cells obtained after 20 days of culturing at a seeding density of 10 cells/cm² (referential example).
FIG. 7A is a microscopic image of cells obtained after 3 days of production culture (comparative example).
FIG. 7B is a microscopic image of cells obtained after 3 days of production culture (comparative example).
FIG. 8A is a microscopic image of cells obtained after 3 days of production culture (example of the present invention).
FIG. 8B is a microscopic image of cells obtained after 3 days of production culture (example of the present invention).
FIG. 9 is a diagram schematically showing a culture process performed in Example 3.
FIG. 10 is a graph showing the result of quantification of a cytokine.
FIG. 11 is a graph showing the result of quantification of a cytokine.
FIG. 12 is a graph showing the result of quantification of a cytokine.
FIG. 13 is a graph showing the positive rates of the cell surface markers of umbilical cord-derived mesenchymal stem cells cultured in the presence of a laminin fragment.
FIG. 14 is a graph showing the positive rates of the cell surface markers of adipose-derived mesenchymal stem cells cultured in the presence of a laminin fragment.
FIG. 15 is a graph showing the positive rates of the cell surface markers of umbilical cord-derived mesenchymal stem cells cultured in the absence of a laminin fragment.
FIG. 16 is a graph showing the amount of HGF contained in a culture supernatant of umbilical cord-derived mesenchymal stem cells.
FIG. 17 is a graph showing the amount of HGF contained in a culture supernatant of adipose-derived mesenchymal stem cells.
FIG. 18 is a graph showing the amount of MCP-1 contained in a culture supernatant of umbilical cord-derived mesenchymal stem cells.
FIG. 19 is a graph showing the amount of GRO/CXCL1 contained in a culture supernatant of umbilical cord-derived mesenchymal stem cells.
FIG. 20 is a graph showing the amount of fibronectin contained in a culture supernatant of umbilical cord-derived mesenchymal stem cells.
FIG. 21 is a graph showing the amount of PDGF-AA contained in a culture supernatant of umbilical cord-derived mesenchymal stem cells.
FIG. 22 is a graph showing the amount of VEGF contained in a culture supernatant of adipose-derived mesenchymal stem cells.
FIG. 23 is a graph showing the amount of TGF-1b contained in a culture supernatant of umbilical cord-derived mesenchymal stem cells.
FIG. 24 is a graph showing the amount of IL-4 contained in a culture supernatant of umbilical cord-derived mesenchymal stem cells.
FIG. 25 is a graph showing the amount of IL-10 contained in a culture supernatant of umbilical cord-derived mesenchymal stem cells.
FIG. 26 is a graph showing the amount of IL-13 contained in a culture supernatant of umbilical cord-derived mesenchymal stem cells.
FIG. 27 is a graph showing the amount of IL-7 contained in a culture supernatant of umbilical cord-derived mesenchymal stem cells.
FIG. 28 is a graph showing the amount of IL-15 contained in a culture supernatant of adipose-derived mesenchymal stem cells.
FIG. 29 is a graph showing the amount of IL-9 contained in a culture supernatant of umbilical cord-derived mesenchymal stem cells.
FIG. 30 is a graph showing the amount of IL-1α contained in a culture supernatant of umbilical cord-derived mesenchymal stem cells.
FIG. 31 is a graph showing the amount of IL-1β contained in a culture supernatant of umbilical cord-derived mesenchymal stem cells.
FIG. 32 is a graph showing the amount of TNF-α contained in a culture supernatant of umbilical cord-derived mesenchymal stem cells.
FIG. 33 is a graph showing the amount of IL-8 contained in a culture supernatant of umbilical cord-derived mesenchymal stem cells.
FIG. 34 is a graph showing the amount of EOTAXIN contained in a culture supernatant of umbilical cord-derived mesenchymal stem cells.
FIG. 35 is a graph showing the amount of IL-6 contained in a culture supernatant of umbilical cord-derived mesenchymal stem cells.
FIG. 36 is a graph showing the amount of G-CSF contained in a culture supernatant of umbilical cord-derived mesenchymal stem cells.
FIG. 37 is a graph showing the amount of GM-CSF contained in a culture supernatant of umbilical cord-derived mesenchymal stem cells.
FIG. 38 is a graph showing the amount of MCP-3 contained in a culture supernatant of umbilical cord-derived mesenchymal stem cells.
FIG. 39 is a graph showing the amount of IL-12P40 contained in a culture supernatant of umbilical cord-derived mesenchymal stem cells.
FIG. 40 is a graph showing the amount of IP-10 contained in a culture supernatant of umbilical cord-derived mesenchymal stem cells.
FIG. 41 is a graph showing the amount of MIP-1α contained in a culture supernatant of umbilical cord-derived mesenchymal stem cells.
FIG. 42 is a graph showing the amount of exosome marker protein contained in a culture supernatant of umbilical cord-derived mesenchymal stem cells.
FIG. 43 is a graph showing a blood flow ratio of the lower limb.

### DETAILED DESCRIPTION

Hereinafter, the present invention will be described in detail; however, the following description is intended to detail the present invention and is not intended to limit the present invention.

In the case of proliferating cells through subculture, the lower the cell density at the time of seeding is, in the larger number of culture vessels the cells can be cultured in the next passage from the cells in a single culture vessel. If the so-called split ratio is 1 : 10, cells that have become confluent in one culture vessel can be seeded in ten culture vessels, and if the split ratio is 1 : 100, cells that have become confluent in one culture vessel can be seeded in a hundred culture vessels. However, a general split ratio of stem cells is from about 1 : 5 to about 1 : 20, and stem cells are rarely seeded at a low cell density so as to have a split ratio of, for example, 1 : 100 or 1 : 1000. In practice, stem cells are seeded at a cell density of greater than 2000 cells/cm² and subcultured.

On the other hand, a study of culturing human mesenchymal stem cells to produce cell-derived components (hereinafter also referred to as "cell products") such as various cytokines and exosomes and administering them to humans for the treatment of diseases, has been conducted. In this case, it is undesirable to culture stem cells in a culture medium containing an animal-derived component, human serum, or a recombinant protein such as an exogenous cytokine or insulin, and purify the obtained supernatant component for their use. This is due to a concern about the risk of the aforementioned exogenous components other than cell-derived components entering a human body. Therefore, it is desirable to use a cell culture supernatant obtained by culturing stem cells in a protein-free culture medium free of exogenous components for therapy.

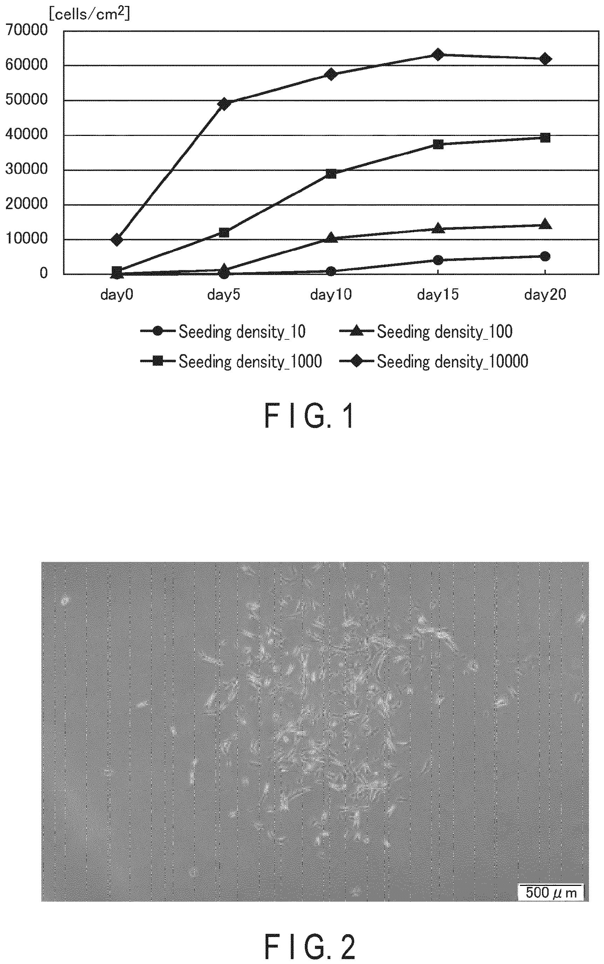

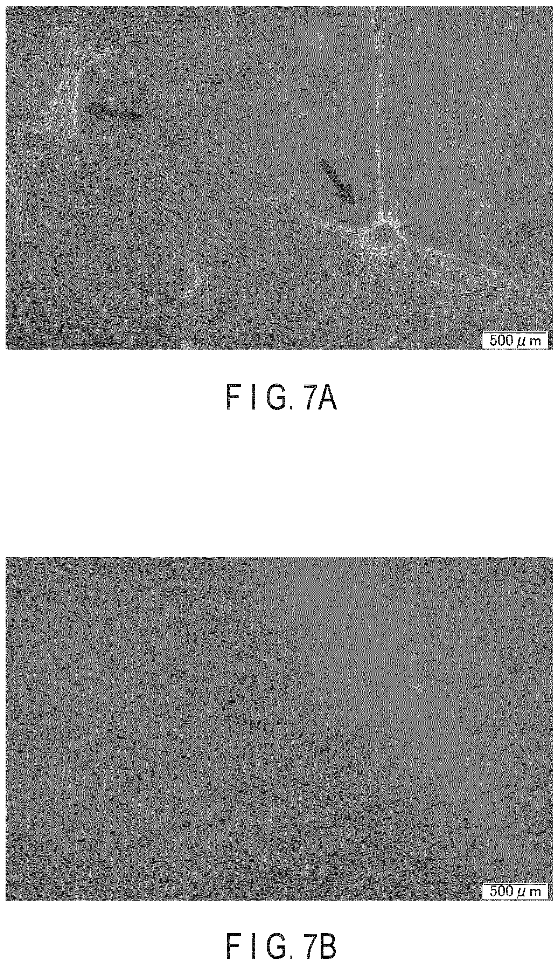

Under such a technical background, when the inventors of the present invention seeded stem cells at a cell density of 10000 cells/cm² and cultured them, the cells were successfully proliferated to a confluent state; however, when the inventors seeded stem cells at a low cell density of 1000 cells/cm² or less and cultured them, the cells stopped proliferating and could not be proliferated to a confluent state (see Example 1 described later and FIGS. 1 and 2). When the inventors seeded stem cells at a cell density of 1000 cells/cm² and cultured them, then cultured the obtained proliferated cells in a protein-free culture medium free of exogenous components (i.e., performing production culture), the cells peeled off from the culture vessel in the middle of the production culture, and the adhered state of the proliferated cells could not be maintained (see Example 2 described later and FIGS. 7A and 7B).

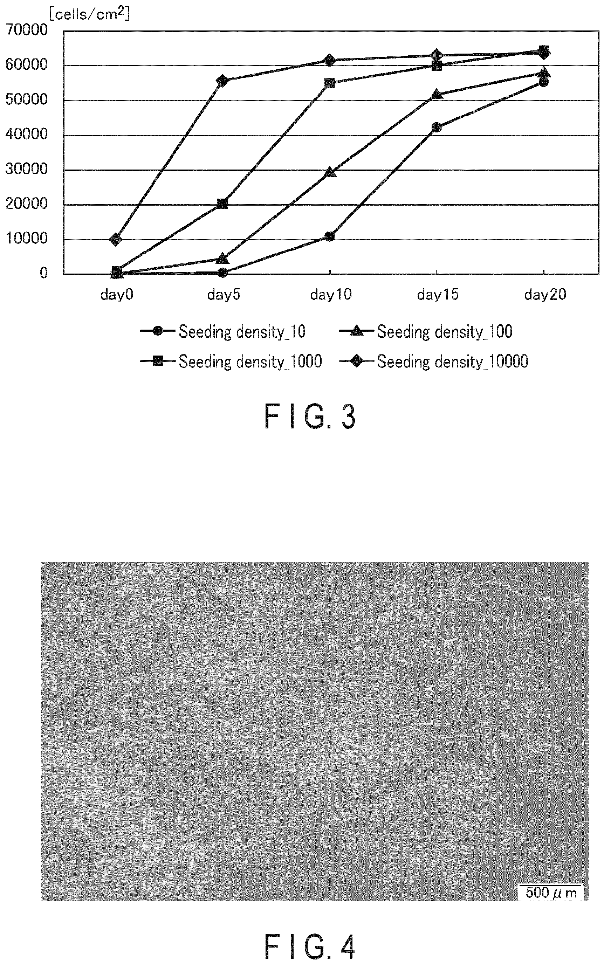

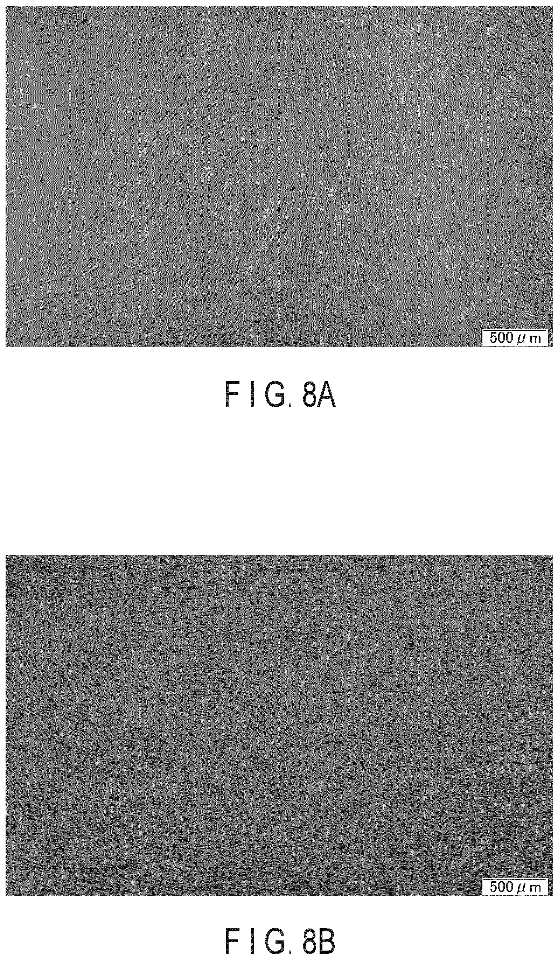

The inventors of the present invention endeavored to solve these problems. As a result, the inventors firstly found that even when stem cells are seeded at a low cell density, they can be proliferated to a confluent state when cultured in the presence of a laminin fragment having integrin-binding activity (hereinafter, also simply referred to as "a laminin fragment") (see Example 1 described later and FIGS. 3 and 4). The inventors secondly found that when stem cells are cultured and proliferated in the presence of a laminin fragment, even when the obtained proliferated cells are thereafter cultured in a protein-free culture medium free of exogenous components, the cells do not peel off from the culture vessel in the middle of culturing, and the adhered state of the proliferated cells can be maintained (see Example 2 described later and FIGS. 8A and 8B). Thirdly, when the inventors cultured the above-described proliferated cells in a protein-free culture medium free of exogenous components (i.e., performed production culture), then further cultured the cells in a culture medium for cell proliferation (i.e., performing recovery culture), and then performed the production culture again, they found that the production culture can be repeated while maintaining the adhered state of the proliferated cells, and that cell-derived components can be produced over a long period of time (see Example 3 described later).

Based on these findings, the inventors completed the present invention. Hereinafter, the methods of the present invention, that is, the "method of producing proliferated cells" and the "method of producing a cell product", will be described in the mentioned order.

### <1. Method of Producing Proliferated Cells>

According to an aspect, there is provided a method of producing proliferated cells, the method including: culturing cells, which have been seeded at a cell density of 0.002 to 2000 cells/cm², through adhesion culture in a proliferation culture medium in the presence of a culture substrate selected from a laminin fragment having integrin-binding activity and a modified form thereof, thereby proliferating the cells.

Preferably, the culturing can be performed in a proliferation culture medium containing the culture substrate (i.e., a laminin fragment having integrin-binding activity or a modified form thereof). Specifically, according to a preferred embodiment, there is provided a method of producing proliferated cells, the method including: culturing cells, which have been seeded at a cell density of 0.002 to 2000 cells/cm², through adhesion culture in a proliferation culture medium containing a laminin fragment having integrin-binding activity (hereinafter, also simply referred to as "a laminin fragment") or a modified form thereof, thereby proliferating the cells.

According to a specific example, the method of producing proliferated cells may include:
preparing a proliferation culture medium containing a laminin fragment or a modified form thereof in advance, suspending cells in the prepared proliferation culture medium such that a seeding density becomes 0.002 to 2000 cells/cm², and seeding the obtained cell suspension in a culture vessel; and
culturing the cells in the proliferation culture medium through adhesion culture, thereby proliferating the cells.

Alternatively, according to another specific example, the method of producing proliferated cells may include:
suspending cells in a proliferation culture medium such that a seeding density becomes 0.002 to 2000 cells/cm², adding a laminin fragment or a modified form thereof to the obtained cell suspension, and seeding the resulting cell suspension in a culture vessel; and
culturing the cells in the proliferation culture medium through adhesion culture, thereby proliferating the cells.

Alternatively, according to another specific example, the method of producing proliferated cells may include:
suspending cells in a proliferation culture medium such that a seeding density becomes 0.002 to 2000 cells/cm², seeding the obtained cell suspension in a culture vessel, and adding a laminin fragment or a modified form thereof to the seeded cell suspension; and
culturing the cells in the proliferation culture medium through adhesion culture, thereby proliferating the cells.

### (Cells)

As the cells, any cells such as stem cells can be used. The cells are preferably mesenchymal stem cells, induced pluripotent stem cells (iPS cells), or embryonic stem cells (ES cells), and more preferably mesenchymal stem cells. The cells are more preferably umbilical cord-derived mesenchymal stem cells, bone marrow-derived mesenchymal stem cells, adipose-derived mesenchymal stem cells, placenta-derived mesenchymal stem cells, or umbilical cord blood-derived mesenchymal stem cells, and still more preferably umbilical cord-derived mesenchymal stem cells.

In a preferred embodiment, the cells are human cells. Specifically, the cells are, for example, human stem cells. The cells are preferably human mesenchymal stem cells, human induced pluripotent stem cells (iPS cells), or human embryonic stem cells (ES cells), and more preferably human mesenchymal stem cells. The cells are more preferably human umbilical cord-derived mesenchymal stem cells, human bone marrow-derived mesenchymal stem cells, human adipose-derived mesenchymal stem cells, human placenta-derived mesenchymal stem cells, or human umbilical cord blood-derived mesenchymal stem cells, and still more preferably human umbilical cord-derived mesenchymal stem cells.

The cells may be frozen cells. Specifically, the cells may be those prepared by thawing frozen cells. By using cryopreserved cells as starting cells to proliferate the cells according to the method of the present invention when it is desired to produce proliferated cells or cell-derived components, proliferated cells or cell-derived components can be produced when necessary.

### (Seeding)

In this method, cells are seeded in a proliferation culture medium at a cell density of 0.002 to 2000 cells/cm².

The cells are seeded at a cell density of preferably 0.002 to 1900 cells/cm², more preferably 0.002 to 1800 cells/cm², still more preferably 0.002 to 1700 cells/cm², still more preferably 0.002 to 1600 cells/cm², still more preferably 0.002 to 1500 cells/cm², still more preferably 0.002 to 1400 cells/cm², still more preferably 0.002 to 1300 cells/cm², still more preferably 0.002 to 1200 cells/cm², still more preferably 0.002 to 1100 cells/cm², still more preferably 0.002 to 1000 cells/cm², still more preferably 0.002 to 900 cells/cm², still more preferably 0.002 to 800 cells/cm², still more preferably 0.002 to 700 cells/cm², still more preferably 0.002 to 600 cells/cm², still more preferably 0.002 to 500 cells/cm², still more preferably 0.002 to 400 cells/cm², still more preferably 0.002 to 300 cells/cm², still more preferably 0.002 to 200 cells/cm², and still more preferably 0.002 to 100 cells/cm².

In consideration of the time needed for cells to reach a confluent state, the cells are seeded at a cell density of preferably 1 to 2000 cells/cm², more preferably 1 to 1900 cells/cm², still more preferably 1 to 1800 cells/cm², still more preferably 1 to 1700 cells/cm², still more preferably 1 to 1600 cells/cm², still more preferably 1 to 1500 cells/cm², still more preferably 1 to 1400 cells/cm², still more preferably 1 to 1300 cells/cm², still more preferably 1 to 1200 cells/cm², still more preferably 1 to 1100 cells/cm², still more preferably 1 to 1000 cells/cm², still more preferably 1 to 900 cells/cm², still more preferably 1 to 800 cells/cm², still more preferably 1 to 700 cells/cm², still more preferably 1 to 600 cells/cm², still more preferably 1 to 500 cells/cm², still more preferably 1 to 400 cells/cm², still more preferably 1 to 300 cells/cm², still more preferably 1 to 200 cells/cm², and still more preferably 1 to 100 cells/cm².

The above cell densities are lower than the seeding density usually employed when culturing and proliferating stem cells. Cells are usually seeded in a single-cell state. Cells in a single-cell state can be prepared by treating a cell mass with a proteolytic enzyme (such as trypsin).

As the proliferation culture medium, a culture medium known as a cell proliferation culture medium can be used depending on the type of cells. For example, in the case of human stem cells, a culture medium commercially available as a culture medium for proliferation of human stem cells can be used.

Considering that the cultured cells and the culture medium are used for therapeutic applications such as treatment of diseases and regenerative medicine, the proliferation culture medium is preferably a serum-free culture medium free of xenogeneic components (xeno-free culture medium).

The proliferation culture medium preferably contains a protein that promotes cell proliferation, whereas a "production culture medium" described later is preferably free of proteins. Examples of the protein that promotes proliferation of stem cells include bFGF (basic fibroblast growth factor), TGFβ1 (transforming growth factor β1), EGF (epidermal growth factor), IGF (insulin-like growth factor), VEGF (vascular endothelial growth factor), HGF (hepatocyte growth factor), insulin, albumin, and transferrin. More specifically, a culture medium prepared by adding a growth factor to a basal medium for cell culture (such as MEM, DMEM, IMDM, Ham's F-12, DMEM/F12, RPMI1640, or the like) may be used as the proliferation culture medium.

In the case of human mesenchymal stem cells, for example, MSC Expansion XSFM B medium (FUJIFILM Wako Pure Chemical Corporation), Mesenchymal Stem Cell Growth Medium DXF (Takara Bio Inc.), MSC NutriStem XF Medium (Biological Industries, Inc.), or the like can be used as the proliferation culture medium. All of these proliferation culture media are serum-free culture media free of xenogeneic components (xeno-free culture media).

As the culture vessel, any vessel used for adhesion culture of cells can be used. Generally, a flat-bottom vessel such as a culture flask, a culture dish, a culture plate or the like can be used as the culture vessel. When a culture vessel having a large bottom area is used, large amounts of proliferated cells and large amounts of cell-derived components can be produced from a single culture vessel. For example, it is desirable to use a culture vessel having a bottom area of 500 cm² or more, preferably a bottom area of 500 to 10000 cm².

### (Proliferation Culture)

The cells seeded at a cell density of 0.002 to 2000 cells/cm² are cultured by adhesion culture in a proliferation culture medium in the presence of a laminin fragment having integrin-binding activity or a modified form thereof.

As described above, in the present invention, when stem cells are cultured in the presence of "a laminin fragment having integrin-binding activity (hereinafter, also simply referred to as "a laminin fragment") or a modified form thereof", the stem cells can be proliferated to a confluent state even when seeded at a low cell density (see Example 1 described later and FIGS. 3 and 4).

Therefore, it is desirable to perform culturing until the cells reach a confluent state. The term "confluent state" as used herein refers to a state in which the cells cover 80% or more of the bottom area of the culture vessel. More preferably, culturing can be performed until the cells reach a state of covering 80 to 90% of the bottom area of the culture vessel.

Culturing in the presence of a laminin fragment or a modified form thereof may be performed by using a proliferation culture medium containing a laminin fragment or a modified form thereof, or using a culture vessel pre-coated with a laminin fragment or a modified form thereof. Culturing using a proliferation culture medium containing a laminin fragment or a modified form thereof is preferred because the amount of a laminin fragment or a modified form thereof used is small and the operation is simple, as compared with culturing using a culture vessel pre-coated with a laminin fragment or a modified form thereof.

Therefore, culturing can be preferably performed in a proliferation culture medium containing a laminin fragment having integrin-binding activity or a modified form thereof.

As the laminin fragment, a laminin fragment known to have integrin-binding activity can be used. It has been reported that when a culture vessel is pre-coated with "a laminin fragment having integrin-binding activity", human ES cells and human iPS cells can be cultured without using feeder cells (for example, Nakagawa et al., Scientific Reports 4, Article Number: 3594 (2014) and International Publication No. 2011/043405). Culturing without using feeder cells, referred to as "feeder-free culture", is widely used because it does not involve a xenogeneic component which is animal-derived. Therefore, a laminin fragment used when proliferating human ES cells or human iPS cells through feeder-free culture in this technical field can also be used in the methods of the present invention.

The laminin fragment is preferably a human-derived laminin fragment since the proliferation culture medium is preferably free of xenogeneic components.

The laminin fragment is preferably a laminin E8 fragment, more preferably a laminin 511 E8 fragment. A laminin 511 E8 fragment is commercially available from Nippi, Incorporated under the trade name iMatrix-511, which can be suitably used. Laminin is composed of three subunit chains, an α chain, a β chain, and a γ chain; and five types of α chains, α1 to α5, three types of β chains, β1 to β3, and three types of γ chains, γ1 to γ3, are known. Laminin 511 refers to a laminin composed of α5, β1, and γ1.

Examples of other laminin fragments that can be used include a laminin 521 E8 fragment, a laminin 411 E8 fragment, a laminin 421 E8 fragment, a laminin 332 E8 fragment, a laminin 311 E8 fragment, a laminin 321 E8 fragment, a laminin 211 E8 fragment, a laminin 221 E8 fragment, a laminin 213 E8 fragment, a laminin 111 E8 fragment, and a laminin 121 E8 fragment.

As the modified form of a laminin fragment, a known complex composed of a laminin fragment having integrin-binding activity and another functional molecule can be used, such as a complex of a laminin fragment having integrin-binding activity and a cell adhesion molecule or a complex of a laminin fragment having integrin-binding activity and a growth factor-binding molecule (see WO2012/137970, WO2014/103534, and WO2016/010082).

Preferably, a complex of a laminin fragment having integrin-binding activity and a growth factor-binding molecule can be used as the modified form of a laminin fragment. The laminin fragment included in the complex can be the laminin fragments described above. The growth factor-binding molecule included in the complex is preferably heparan sulfate. The complex has growth factor-binding activity in addition to integrin-binding activity.

The modified form of a laminin fragment is more preferably a complex of a laminin E8 fragment and a growth factor-binding molecule. The laminin E8 fragment included in the complex is preferably the laminin E8 fragment described above. The growth factor-binding molecule included in the complex is preferably heparan sulfate. Therefore, the modified form of a laminin fragment is more preferably a complex of the above-described laminin E8 fragment and heparan sulfate. The modified form of a laminin fragment is most preferably a complex of the laminin 511 E8 fragment and heparan sulfate or a complex of the laminin 421 E8 fragment and heparan sulfate.

The complex of a laminin fragment having integrin-binding activity and a growth factor-binding molecule can be produced as a recombinant protein by using known genetic recombination technology.

For example, the concentration of a laminin fragment or a modified form thereof in the proliferation culture medium can be 0.005 µg to 2 µg per 1 cm² culture area of the culture vessel. Preferably, the concentration of a laminin fragment or a modified form thereof in the proliferation culture medium can be 0.01 µg to 0.5 µg per 1 cm² culture area of the culture vessel. More preferably, the concentration of a laminin fragment or a modified form thereof in the proliferation culture medium can be 0.05 µg to 0.25 µg per 1 cm² culture area of the culture vessel.

Here, assuming that the liquid amount of the proliferation culture medium is, for example, 200 µl/cm² (culture area), 0.005 µg/cm² to 2 µg/cm² corresponds to 0.025 µg/ml to 10 µg/ml; 0.01 µg/cm² to 0.5 µg/cm² corresponds to 0.05 µg/ml to 2.5 µg/ml; and 0.05 µg/cm² to 0.25 µg/cm² corresponds to 0.25 µg/ml to 1.25 µg/ml.

As described above, culturing can be performed in any culture vessel, and can, for example, be performed in a culture vessel having a bottom area of, for example, 500 cm² or more, preferably 500 to 10000 cm². In the middle of culturing, the proliferation culture medium may be appropriately replaced with a new culture medium having the same composition.

### (Effects)

Conventionally, when cells are seeded at a low cell density, they stop proliferating and cannot proliferate to a confluent state. On the other hand, the method described above enables cells seeded at a low cell density to be proliferated at a high proliferation ratio. Accordingly, it is possible to increase the number of culture vessels that can be cultured in the next passage from the cells in a single culture vessel, allowing for efficient production of the proliferated cells and cell-derived components.

### <2. Method of Producing Cell Product>

### <2-1. First Embodiment (Embodiment In Which Production Culture Step Is Performed Single Time)>

According to another aspect, there is provided a method of producing a cell product, the method including:
culturing cells in a proliferation culture medium according to the above-described "method of producing proliferated cells", thereby obtaining proliferated cells in an adhered state; and
culturing the proliferated cells in a production culture medium while maintaining the adhered state, thereby causing the cells to produce a cell product.

Specifically, the method of producing a cell product includes:
culturing cells, which have been seeded at a cell density of 0.002 to 2000 cells/cm², through adhesion culture in a proliferation culture medium in the presence of a culture substrate selected from a laminin fragment having integrin-binding activity and a modified form thereof, thereby obtaining proliferated cells in an adhered state; and
culturing the proliferated cells in a production culture medium while maintaining the adhered state, thereby causing the cells to produce a cell product.

In the description provided below, the culturing performed in the proliferation culture medium will be referred to as "proliferation culture" and the culturing performed in the production culture medium will be referred to as "production culture".

As described above, the proliferation culture can preferably be performed in a proliferation culture medium containing a laminin fragment having integrin-binding activity or a modified form thereof. Specifically, according to a preferred embodiment, there is provided a method of producing a cell product, the method including:
culturing cells, which have been seeded at a cell density of 0.002 to 2000 cells/cm², through adhesion culture in a proliferation culture medium containing a laminin fragment having integrin-binding activity or a modified form thereof, thereby obtaining proliferated cells in an adhered state; and
culturing the proliferated cells in a production culture medium while maintaining the adhered state, thereby causing the cells to produce a cell product.

### (Proliferation Culture)

The steps up to the proliferation culture can be performed as described in <1. Method of Producing Proliferated Cells>. Thus, proliferated cells can be obtained in the adhered state. By performing the proliferation culture until the cells reach a confluent state, proliferated cells can be obtained in a confluent state.

### (Production Culture)

After the proliferation culture is performed, the proliferated cells are cultured in a production culture medium while maintaining the adhered state. The phrase "culturing the proliferated cells in a production culture medium while maintaining the adhered state" means that after the proliferation culture is performed, the cells adhered to the bottom surface of the culture vessel are subjected to production culture while maintaining the adhered state without peeling off from the bottom surface of the culture vessel.

The production culture can be performed by replacing the proliferation culture medium in the culture vessel with a production culture medium after the proliferation culture is performed. Thus, the proliferated cells can be cultured in the production culture medium while maintaining the adhered state.

During the production culture, the cells can produce a cell product and release it into the production culture medium. The cell product is any substance that cells release into the production culture medium, and examples of it include: cellular metabolites such as amino acids, lipids, and saccharides; hormones; peptides; secreted proteins such as cytokines and extracellular matrices; and exosomes. For example, stem cells can produce various cytokines and exosomes and release them into the production culture medium. Thus, the method of producing a cell product may further include the step of collecting a supernatant of the production culture medium after performing the production culture.

The supernatant of the production culture medium obtained in the collection step is assumed to be used for therapeutic applications such as treatment of diseases and regenerative medicine. Therefore, the production culture medium is preferably a culture medium free of xenogeneic components. When the cells to be cultured are human cells, the xenogeneic components refer to components derived from animals not humans. The production culture medium is also preferably a culture medium free of cytokines or insulin. The production culture medium is also preferably a protein-free culture medium. The production culture medium is also preferably a serum-free culture medium.

More preferably, the production culture medium is a cell culture medium free of xenogeneic components and free of cytokines and insulin. Still more preferably, the production culture medium is a cell culture medium free of xenogeneic components, free of cytokines and insulin, and free of proteins. Still more preferably, the production culture medium is a cell culture medium free of xenogeneic components, free of cytokines and insulin, free of proteins, and free of serum.

Among cell culture media suitable for the types of cells, a cell culture medium free of the above-described components (i.e., xenogeneic components, cytokines, insulin, proteins, and human serum) can be used as the production culture medium. More specifically, a basal medium for cell culture (e.g., MEM, DMEM, IMDM, Ham's F-12, DMEM/F12, RPMI1640, etc.) or a basal medium for cell culture supplemented with nutrient components for cells may be used as the production culture medium. The basal media for cell culture are commercially available and generally include amino acids, vitamins, inorganic salts and a carbon source. The production culture medium need not contain laminin fragments or modified forms thereof.

For example, in the case of human mesenchymal stem cells, DMEM/F12 medium supplemented with amino acids can be used as the production culture medium. As the amino acids to be added, a commercially available amino acid solution for addition to a culture medium can be used, such as a MEM essential amino acid solution (FUJIFILM Wako Pure Chemical Corporation), a MEM non-essential amino acid solution (FUJIFILM Wako Pure Chemical Corporation), or the like. The production culture medium is free of all of xenogeneic components, cytokines, insulin, proteins, and human serum.

The production culture period is not particularly limited, and can be, for example, 0.5 to 10 days, and preferably 2 to 5 days.

### (Effects)

Conventionally, cells stop proliferating in the middle of the proliferation culture, or cells peel off from a culture vessel in the middle of the production culture and cannot maintain the adhered state. On the other hand, according to the method described above, cells seeded at a low cell density can be proliferated at a high proliferation ratio, and the production culture can thereafter be performed while maintaining the adhered state of the obtained proliferated cells. Thus, large amounts of cell products can be produced via a simple method.

Specifically, since the above-described method allows cells seeded at a low cell density to be proliferated at a high proliferation ratio, large amounts of cell products can be produced from small amounts of cells as a raw material. In addition, the above-described method is simple because it can maintain the adhered state of the cells and thus allows for a transition from the proliferation culture to the production culture merely through exchanging the culture medium. Namely, the above-described method does not require subculturing, thereby eliminating lot differences due to subculturing and costs associated with subculturing. In addition, the above-described method is simple because it can maintain the adhered state of the cells and can thus easily collect the culture supernatant containing the cell product.

### <2-2. Second Embodiment (Embodiment In Which Production Culture Step is Performed Multiple Times)>

According to another aspect, there is provided a method of producing a cell product, the method including:
culturing cells in a proliferation culture medium according to the above-described "method of producing proliferated cells", thereby obtaining proliferated cells in an adhered state;
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells.

Specifically, the method of producing a cell product includes:
culturing cells, which have been seeded at a cell density of 0.002 to 2000 cells/cm², through adhesion culture in a proliferation culture medium in the presence of a culture substrate selected from a laminin fragment having integrin-binding activity and a modified form thereof, thereby obtaining proliferated cells in an adhered state; and
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells.

In the description provided below, the culturing performed in the proliferation culture medium will be referred to as "proliferation culture", the culturing performed in the production culture medium will be referred to as "production culture", and the culturing performed in the recovery medium will be referred to as "recovery culture".

As described above, the proliferation culture can preferably be performed in a proliferation culture medium containing a laminin fragment having integrin-binding activity or a modified form thereof. Specifically, according to a preferred embodiment, there is provided a method of producing a cell product, the method including:
culturing cells, which have been seeded at a cell density of 0.002 to 2000 cells/cm², through adhesion culture in a proliferation culture medium containing a laminin fragment having integrin-binding activity or a modified form thereof, thereby obtaining proliferated cells in an adhered state; and
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells.

### (Proliferation Culture)

The steps up to the proliferation culture can be performed as described in <1. Method of Producing Proliferated Cells>. Thus, proliferated cells can be obtained in the adhered state. By performing the proliferation culture until the cells reach a confluent state, proliferated cells can be obtained in a confluent state.

### (Production Culture)

The step of the production culture can be performed as described in <2-1. First Embodiment (Embodiment In Which Production Culture Step Is Performed Single Time)>. Thereby, it is possible to cause the cells to produce a cell product.

### (Recovery Culture)

After the production culture is performed, the proliferated cells are cultured in a recovery culture medium while maintaining the adhered state. The phrase "culturing the proliferated cells in a recovery culture medium while maintaining the adhered state" means that after the production culture is performed, the cells adhered to the bottom surface of the culture vessel are subjected to recovery culture while maintaining the adhered state without peeling off from the bottom surface of the culture vessel.

The recovery culture can be performed by replacing the production culture medium in the culture vessel with a recovery culture medium after the production culture is performed. Thus, the proliferated cells can be further cultured in the recovery culture medium while maintaining the adhered state, after being cultured in the production culture medium while maintaining the adhered state.

During the recovery culture, the cells can be recovered again to a state where the cells can produce cell products in sufficient amounts. Namely, the recovery culture is a culture performed to recover the ability of the cells to produce a cell product for the next production culture step.

The recovery culture can be performed using a culture medium used for the proliferation culture of the cells. Namely, as the recovery culture medium, a culture medium known as a cell proliferation culture medium can be used depending on the type of cells. For example, in the case of human stem cells, a culture medium commercially available as a culture medium for proliferation of human stem cells can be used.

Considering that the cultured cells and culture medium are used for therapeutic applications such as treatment of diseases and regenerative medicine, the recovery culture medium is preferably a serum-free culture medium free of xenogeneic components (xeno-free culture medium).

The recovery culture medium preferably contains a protein that promotes cell proliferation, whereas the "production culture medium" is preferably free of a protein. Examples of the protein that promotes proliferation of stem cells include bFGF (basic fibroblast growth factor), TGFβ1 (transforming growth factor β1), EGF (epidermal growth factor), IGF (insulin-like growth factor), VEGF (vascular endothelial growth factor), HGF (hepatocyte growth factor), insulin, albumin, and transferrin. More specifically, a culture medium prepared by adding a growth factor to a basal medium for cell culturing (e.g., MEM, DMEM, IMDM, Ham's F-12, DMEM/F12, RPMI1640, etc.) may be used as the recovery culture medium.

A culture medium having the same composition as that of the proliferation culture medium may be used as the recovery culture medium. However, the recovery culture medium need not contain laminin fragments or modified forms thereof.

In the case of human mesenchymal stem cells, for example, MSC Expansion XSFM B medium (FUJIFILM Wako Pure Chemical Corporation), Mesenchymal Stem Cell Growth Medium DXF (Takara Bio Inc.), MSC NutriStem XF Medium (Biological Industries, Inc.), or the like can be used as the recovery culture medium. All of these recovery culture media are serum-free culture media free of xenogeneic components (xeno-free culture media).

As described above, the production culture and the recovery culture can be alternately repeated while maintaining the adhered state of the cells. A cycle of the production culture and the recovery culture can be repeated without limitation as long as the cells can recover their ability to produce cell products. For example, a cycle of the production culture and the recovery culture can be repeated 2 to 10 times. This allows the cells to produce a cell product and release it into the culture medium each time the production culture is performed. Thus, the method of producing a cell product may further include the step of collecting a supernatant of the production culture medium after performing the production culture.

The period of the recovery culture is not particularly limited, and can be, for example, 0.5 to 10 days, and preferably 2 to 5 days.

### (Effects)

In the method according to the second embodiment, cells seeded at a low cell density can be proliferated at a high proliferation ratio, and the production culture can thereafter be performed while maintaining the adhered state of the obtained proliferated cells, as in the method according to the first embodiment. Also, in the method according to the second embodiment, the recovery culture can be performed while maintaining the adhered state of the cells that have completed the production culture, and thus the ability of the cells to produce cell products can be recovered. Thereby, the production culture step can be repeatedly performed multiple times while maintaining the adhered state of the cells, so that large amounts of cell products can be produced over a long period of time via a simple method.

Specifically, since the method according to the second embodiment allows cells seeded at a low cell density to be proliferated at a high proliferation ratio, large amounts of cell products can be produced from small amounts of cells as a raw material. In addition, the method according to the second embodiment is simple because it can maintain the adhered state of the cells and thus allows for both a transition from the proliferation culture to the production culture and a repetition of a cycle of the production culture and the recovery culture, merely through exchanging the culture medium. Namely, the method according to the second embodiment does not require subculturing, thereby eliminating lot differences due to subculturing and costs associated with subculturing. In addition, the method according to the second embodiment is simple because it can maintain the adhered state of the cells and thus can easily collect the culture supernatant containing the cell product.

In particular, the method according to the second embodiment has an advantage in that it enables production of a cell product over a long period of time because the cell product can be produced each time a cycle of the production culture and the recovery culture is repeated. The method according to the second embodiment also has an advantage in that it enables continuous production of large amounts of cell products because the production amounts of the cell products do not decrease even when a cycle of the production culture and the recovery culture is repeated.

### <3. Preferred Embodiments>

Hereinafter, the preferred embodiments of the present invention will be described.

[A1] A method of producing proliferated cells, the method including: culturing cells, which have been seeded at a cell density of 0.002 to 2000 cells/cm², through adhesion culture in a proliferation culture medium in a presence of a culture substrate selected from a laminin fragment having integrin-binding activity and a modified form thereof, thereby proliferating the cells.

[A2] A method of producing proliferated cells, the method including: culturing cells, which have been seeded at a cell density of 0.002 to 2000 cells/cm², through adhesion culture in a proliferation culture medium containing a culture substrate selected from a laminin fragment having integrin-binding activity and a modified form thereof, thereby proliferating the cells.

[A3] A method of producing proliferated cells, the method including:
seeding, in a culture vessel, a cell suspension containing a proliferation culture medium, cells of such an amount that achieves a seeding density of 0.002 to 2000 cells/cm², and a culture substrate selected from a laminin fragment having integrin-binding activity and a modified form thereof; and thereafter
culturing the cells through adhesion culture in the proliferation culture medium, thereby proliferating the cells.

[A4] A method of producing proliferated cells, the method including:
seeding, in a culture vessel, a cell suspension containing a proliferation culture medium and cells of such an amount that achieves a seeding density of 0.002 to 2000 cells/cm²;
adding, to the seeded cell suspension, a culture substrate selected from a laminin fragment having integrin-binding activity and a modified form thereof; and thereafter
culturing the cells through adhesion culture in the proliferation culture medium, thereby proliferating the cells.

[A5] The method according to any one of [A1] to [A4], wherein the cells are stem cells.

[A6] The method according to any one of [A1] to [A5], wherein the cells are mesenchymal stem cells, induced pluripotent stem cells (iPS cells), or embryonic stem cells (ES cells).

[A7] The method according to any one of [A1] to [A6], wherein the cells are mesenchymal stem cells.

[A8] The method according to any one of [A1] to [A7], wherein the cells are umbilical cord-derived mesenchymal stem cells, bone marrow-derived mesenchymal stem cells, adipose-derived mesenchymal stem cells, placenta-derived mesenchymal stem cells, or umbilical cord blood-derived mesenchymal stem cells.

[A9] The method according to any one of [A1] to [A8], wherein the cells are umbilical cord-derived mesenchymal stem cells.

[A10] The method according to any one of [A1] to [A5], wherein the cells are human stem cells.

[A11] The method according to any one of [A1] to [A6] and [A10], wherein the cells are human mesenchymal stem cells, human induced pluripotent stem cells (iPS cells), or human embryonic stem cells (ES cells).

[A12] The method according to any one of [A1] to [A7], [A10], and [A11], wherein the cells are human mesenchymal stem cells.

[A13] The method according to any one of [A1] to [A8] and [A10] to [A12], wherein the cells are human umbilical cord-derived mesenchymal stem cells, human bone marrow-derived mesenchymal stem cells, human adipose-derived mesenchymal stem cells, human placenta-derived mesenchymal stem cells, or human umbilical cord blood-derived mesenchymal stem cells.

[A14] The method according to any one of [A1] to [A13], wherein the cells are human umbilical cord-derived mesenchymal stem cells.

[A15] The method according to any one of [A1] to [A14], wherein the cells are frozen cells.

[A16] The method according to any one of [A1] to [A15], wherein the cells are prepared by thawing frozen cells.

[A17] The method according to any one of [A1] to [A16], wherein the cells are seeded at a cell density of 0.002 to 1900 cells/cm², preferably 0.002 to 1800 cells/cm², more preferably 0.002 to 1700 cells/cm², still more preferably 0.002 to 1600 cells/cm², still more preferably 0.002 to 1500 cells/cm², still more preferably 0.002 to 1400 cells/cm², still more preferably 0.002 to 1300 cells/cm², still more preferably 0.002 to 1200 cells/cm², still more preferably 0.002 to 1100 cells/cm², still more preferably 0.002 to 1000 cells/cm², still more preferably 0.002 to 900 cells/cm², still more preferably 0.002 to 800 cells/cm², still more preferably 0.002 to 700 cells/cm², still more preferably 0.002 to 600 cells/cm², still more preferably 0.002 to 500 cells/cm², still more preferably 0.002 to 400 cells/cm², still more preferably 0.002 to 300 cells/cm², still more preferably 0.002 to 200 cells/cm², and still more preferably 0.002 to 100 cells/cm².

[A18] The method according to any one of [A1] to [A16], wherein the cells are seeded at a cell density of 1 to 2000 cells/cm², preferably 1 to 1900 cells/cm², more preferably 1 to 1800 cells/cm², still more preferably 1 to 1700 cells/cm², still more preferably 1 to 1600 cells/cm², still more preferably 1 to 1500 cells/cm², still more preferably 1 to 1400 cells/cm², still more preferably 1 to 1300 cells/cm², still more preferably 1 to 1200 cells/cm², still more preferably 1 to 1100 cells/cm², still more preferably 1 to 1000 cells/cm², still more preferably 1 to 900 cells/cm², still more preferably 1 to 800 cells/cm², still more preferably 1 to 700 cells/cm², still more preferably 1 to 600 cells/cm², still more preferably 1 to 500 cells/cm², still more preferably 1 to 400 cells/cm², still more preferably 1 to 300 cells/cm², still more preferably 1 to 200 cells/cm², and still more preferably 1 to 100 cells/cm².

[A19] The method according to any one of [A1] to [A18], wherein the proliferation culture medium is a proliferation culture medium containing a protein that promotes proliferation of the cells.

[A20] The method according to any one of [A1] to [A19], wherein the proliferation culture medium is a culture medium containing a basal medium for cell culture supplemented with a growth factor.

[A21] The method according to any one of [A1] to [A20], wherein the proliferation culture medium is a basal medium for cell culture supplemented with a growth factor.

[A22] The method according to any one of [A1] to [A21], wherein the culturing is performed until the cells reach a confluent state.

[A23] The method according to any one of [A1] to [A22], wherein the culturing is performed in a culture vessel having a bottom area of 500 cm² or more.

[A24] The method according to any one of [A1] to [A23], wherein the culturing is performed in a culture vessel having a bottom area of 500 to 10000 cm².

[A25] The method according to any one of [A1] to [A24], wherein the culture substrate is a laminin fragment having integrin-binding activity.

[A26] The method according to [A25], wherein the laminin fragment is a human-derived laminin fragment.

[A27] The method according to [A25] or [A26], wherein the laminin fragment is a laminin E8 fragment.

[A28] The method according to any one of [A25] to [A27], wherein the laminin fragment is a laminin 511 E8 fragment, a laminin 521 E8 fragment, a laminin 411 E8 fragment, a laminin 421 E8 fragment, a laminin 332 E8 fragment, a laminin 311 E8 fragment, a laminin 321 E8 fragment, a laminin 211 E8 fragment, a laminin 221 E8 fragment, a laminin 213 E8 fragment, a laminin 111 E8 fragment, or a laminin 121 E8 fragment.

[A29] The method according to any one of [A25] to [A28], wherein the laminin fragment is a laminin 511 E8 fragment.

[A30] The method according to any one of [A1] to [A24], wherein the culture substrate is a modified form of a laminin fragment having integrin-binding activity.

[A31] The method according to [A30], wherein the modified form is a complex of a laminin fragment having integrin-binding activity and another functional molecule.

[A32] The method according to [A30] or [A31], wherein the modified form is a complex of a laminin fragment having integrin-binding activity and a growth factor-binding molecule.

[A33] The method according to [A31] or [A32], wherein the laminin fragment is a laminin E8 fragment.

[A34] The method according to [A33], wherein the laminin E8 fragment is a laminin 511 E8 fragment, a laminin 521 E8 fragment, a laminin 411 E8 fragment, a laminin 421 E8 fragment, a laminin 332 E8 fragment, a laminin 311 E8 fragment, a laminin 321 E8 fragment, a laminin 211 E8 fragment, a laminin 221 E8 fragment, a laminin 213 E8 fragment, a laminin 111 E8 fragment, or a laminin 121 E8 fragment.

[A35] The method according to [A33] or [A34], wherein the laminin E8 fragment is a laminin 511 E8 fragment.

[A36] The method according to [A33] or [A34], wherein the laminin E8 fragment is a laminin 421 E8 fragment.

[A37] The method according to any one of [A32] to [A36], wherein the growth factor-binding molecule is heparan sulfate.

[A38] The method according to any one of [A30] to [A37], wherein the modified form is a complex of a laminin 511 E8 fragment and heparan sulfate.

[A39] The method according to any one of [A30] to [A37], wherein the modified form is a complex of a laminin 421 E8 fragment and heparan sulfate.

[A40] The method according to any one of [A1] to [A39], wherein a concentration of the laminin fragment or modified form thereof in the proliferation culture medium is 0.005 µg to 2 µg per 1 cm² culture area of the culture vessel.

[A41] The method according to any one of [A1] to [A40], wherein a concentration of the laminin fragment or modified form thereof in the proliferation culture medium is 0.01 µg to 0.5 µg per 1 cm² culture area of the culture vessel.

[A42] The method according to any one of [A1] to [A41], wherein a concentration of the laminin fragment or modified form thereof in the proliferation culture medium is 0.05 µg to 0.25 µg per 1 cm² culture area of the culture vessel.

[B1] A method of producing a cell product, the method including:
culturing cells in a proliferation culture medium according to the method according to any one of [A1] to [A42], thereby obtaining proliferated cells in an adhered state; and
culturing the proliferated cells in a production culture medium while maintaining the adhered state, thereby causing the cells to produce a cell product.

[B2] A method of producing a cell product, the method including:
culturing cells in a proliferation culture medium according to the method according to any one of [A1] to [A42], thereby obtaining proliferated cells in an adhered state;
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells.

[B3] The method according to [B1] or [B2], wherein the cell product is: a cellular metabolite such as an amino acid, lipid, or saccharide; a hormone; a peptide; a secreted protein such as a cytokine or an extracellular matrix; or an exosome.

[B4] The method according to any one of [B1] to [B3], wherein the cell product is a cytokine.

[B5] The method according to any one of [B1] to [B3], wherein the cell product is an exosome.

[B6] The method according to any one of [B1] to [B5], wherein the production culture medium is a culture medium free of a xenogeneic component.

[B7] The method according to any one of [B1] to [B6], wherein the production culture medium is a culture medium free of a cytokine or insulin.

[B8] The method according to any one of [B1] to [B7], wherein the production culture medium is a protein-free culture medium.

[B9] The method according to any one of [B1] to [B8], wherein the production culture medium is a serum-free culture medium.

[B10] The method according to any one of [B1] to [B9], further including collecting a supernatant of the production culture medium after performing the culturing in the production culture medium.

[B11] The method according to any one of [B1] to [B10], wherein the production culture medium is a culture medium containing a basal medium for cell culture, or a culture medium containing a basal medium for cell culture supplemented with a nutrient component for the cells.

[B12] The method according to any one of [B1] to [B11], wherein the production culture medium is a basal medium for cell culture, or a basal medium for cell culture supplemented with a nutrient component for the cells.

[B13] The method according to any one of [B1] to [B12], wherein the production culture medium is a basal medium for cell culture supplemented with a nutrient component for the cells, preferably an amino acid.

[B14] The method according to any one of [B1] to [B13], wherein the production culture medium is free of a laminin fragment or a modified form thereof.

[B15] The method according to any one of [B1] to [B14], wherein the culturing in the production culture medium is performed for 0.5 to 10 days, preferably 2 to 5 days.

[B16] The method according to any one of [B2] to [B15], wherein the recovery culture medium is a proliferation culture medium of the cells.

[B17] The method according to any one of [B2] to [B16], wherein the recovery culture medium is a culture medium containing a protein that promotes proliferation of the cells.

[B18] The method according to any one of [B2] to [B17], wherein the recovery culture medium is a culture medium containing a basal medium for cell culture supplemented with a growth factor.

[B19] The method according to any one of [B2] to [B18], wherein the recovery culture medium is a basal medium for cell culture supplemented with a growth factor.

[B20] The method according to any one of [B2] to [B19], wherein the recovery culture medium has a composition same as a composition of the proliferation culture medium.

[B21] The method according to any one of [B2] to [B20], wherein the recovery culture medium is free of a laminin fragment or a modified form thereof.

[B22] The method according to any one of [B2] to [B21], wherein the culturing in the recovery culture medium is performed for 0.5 to 10 days, preferably 2 to 5 days.

[B23] The method according to any one of [B2] to [B22], wherein a cycle of the culturing performed in the production culture medium and the culturing performed in the recovery culture medium is repeated 2 to 10 times.

Hereinafter, "a mesenchymal stem cell population and a method of producing the same", "a culture supernatant of stem cells and a method of producing the same", and "a therapeutic agent containing a mesenchymal stem cell population or a culture supernatant of stem cells" will be described.

### <4. Mesenchymal Stem Cell Population and Method of Producing Same>

The inventors of the present application made a new discovery that the expression of HLA-ABC and CD105, which are cell surface markers of mesenchymal stem cells, decreases when mesenchymal stem cells are cultured in a proliferation culture medium in the presence of a laminin fragment having integrin-binding activity according to the method described in <1. Method of Producing Proliferated Cells> (see Example 4 described later).

It is known in this technical field that mesenchymal stem cells are HLA-ABC positive and CD105 positive. Also, in this technical field, the fact that a cell surface marker is positive can be represented by a value of the positive rate of the cell surface marker. The positive rate of the cell surface marker can be determined by a flow cytometer using a fluorescence-labeled antibody of the cell surface marker, as described in the examples below.

Therefore, according to an embodiment, there is provided a method of producing a mesenchymal stem cell population having a reduced HLA-ABC positive rate, the method including: culturing mesenchymal stem cells in a proliferation culture medium in the presence of a culture substrate selected from a laminin fragment having integrin-binding activity and a modified form thereof, thereby obtaining proliferated cells. In this method, the obtained proliferated cells have a reduced HLA-ABC positive rate. The "HLA-ABC positive rate" represents the ratio (%) of HLA-ABC positive mesenchymal stem cells in the mesenchymal stem cell population.

According to this method, a mesenchymal stem cell population having a reduced HLA-ABC positive rate as compared to the mesenchymal stem cell population before the culturing can be obtained. For example, according to this method, a mesenchymal stem cell population having an HLA-ABC positive rate of 70% or less can be obtained. In this mesenchymal stem cell population, the HLA-ABC positive rate is preferably 60% or less, more preferably 50% or less, still more preferably 40% or less, still more preferably 30% or less, still more preferably 20% or less, and still more preferably 10% or less.

According to a preferred embodiment, there is provided a method of producing a mesenchymal stem cell population having a reduced HLA-ABC positive rate and a reduced CD105 positive rate, the method including: culturing mesenchymal stem cells in a proliferation culture medium in the presence of a culture substrate selected from a laminin fragment having integrin-binding activity and a modified form thereof, thereby obtaining proliferated cells. In this method, the obtained proliferated cells have a reduced HLA-ABC positive rate and a reduced CD105 positive rate. The "HLA-ABC positive rate" represents the ratio (%) of HLA-ABC positive mesenchymal stem cells in the mesenchymal stem cell population, and the "CD105 positive rate" represents the ratio (%) of CD-105 positive mesenchymal stem cells in the mesenchymal stem cell population.

According to this method, a mesenchymal stem cell population having a reduced HLA-ABC positive rate and a reduced CD105 positive rate as compared to the mesenchymal stem cell population before the culturing can be obtained. For example, according to this method, a mesenchymal stem cell population having an HLA-ABC positive rate of 70% or less and a CD105 positive rate of 50% or less can be obtained. In this mesenchymal stem cell population, the HLA-ABC positive rate is preferably 60% or less, more preferably 50% or less, still more preferably 40% or less, still more preferably 30% or less, still more preferably 20% or less, and still more preferably 10% or less. In this mesenchymal stem cell population, the CD105 positive rate is preferably 40% or less, more preferably 30% or less, still more preferably 20% or less, and still more preferably 10% or less.

The above-described "method of producing a mesenchymal stem cell population" can be performed as described in <1. Method of Producing Proliferated Cells>. The section <1. Method of Producing Proliferated Cells> describes the fact that the cell density at the start of culturing (i.e., seeding density of the cells) adopts a seeding density lower than usual (0.002 to 2000 cells/cm²). However, in the "method of producing a mesenchymal stem cell population", a seeding density lower than usual need not be adopted, and a seeding density exceeding 2000 cells/cm² may be adopted. Namely, in the "method of producing a mesenchymal stem cell population", the cell density at the start of proliferation culture (i.e., seeding density of the cells) can be, for example, 2001 to 1000000 cells/cm², and preferably 5000 to 20000 cells/cm². Needless to say, a seeding density lower than usual (0.002 to 2000 cells/cm²) may be employed in this method.

Mesenchymal stem cells having a low HLA-ABC positive rate are less likely to encounter an immune response (immune rejection) of the host when administered as a therapeutic agent, and can thus be expected to have a high cell survival rate and exert high therapeutic effects.

It has been reported that cells having a low CD105 positive rate have a high expression level in TGFb1 and suppress T-cell proliferation, and are thus expected to have therapeutic effects on inflammatory diseases.

The term "mesenchymal stem cells" as used herein includes both mesenchymal stem cells before cultured by the above-described method and mesenchymal stem cells obtained by performing culturing in the above-described method. Namely, the term "mesenchymal stem cells" as used herein also includes HLA-ABC negative mesenchymal stem cells and CD105 negative mesenchymal stem cells in addition to HLA-ABC positive mesenchymal stem cells and CD105 positive mesenchymal stem cells. Therefore, the term "mesenchymal stem cells" as used herein can be defined as mesenchymal stem cells positive for CD44, CD73, and CD90 and negative for CD45, CD34, CD31, and HLA-DR.

### <5. Culture Supernatant of Stem Cells and Method of Producing Same>

The inventors of the present application made a new discovery that when mesenchymal stem cells are cultured in a proliferation culture medium in the presence of a laminin fragment having integrin-binding activity, and then the culturing performed in a production culture medium and the culturing performed in a recovery culture medium are alternately repeated, followed by the collecting of a supernatant of the production culture medium, according to the method described in <2. Method of Producing Cell Product>, the amounts of cytokines, extracellular matrix, and exosomes in the culture supernatant increase as the number of times of the production culture increases (see Example 5 described below).

Thus, according to an embodiment, there is provided a method of producing a culture supernatant of stem cells, the method including:
culturing stem cells through adhesion culture in a proliferation culture medium in the presence of a culture substrate selected from a laminin fragment having integrin-binding activity and a modified form thereof, thereby obtaining proliferated cells in an adhered state; and
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells, and the method further includes collecting a supernatant of the production culture medium after performing the culturing in the production culture medium. In this method, the stem cells are, for example, mesenchymal stem cells.

The expression "culture supernatant of cells" as used herein refers to a supernatant liquid obtained by culturing cells in a culture medium and removing the cells and impurities from a mixture of the cells and the culture medium obtained after culturing. It can also be referred to as "a cell-derived culture supernatant". The culture supernatant of cells also includes a culture supernatant subjected to treatment such as sterilization treatment. "A culture supernatant of specific cells" such as "a culture supernatant of stem cells" and "a culture supernatant of mesenchymal stem cells" also have the same meaning.

This method can be performed as described in <2. Method of Producing Cell Product>. The section <2. Method of Producing Cell Product> describes the fact that the cell density at the start of proliferation culture (i.e., seeding density of the cells) adopts a seeding density lower than usual (0.002 to 2000 cells/cm²). However, in the "method of producing a culture supernatant of stem cells", a seeding density lower than usual need not be adopted, and a seeding density exceeding 2000 cells/cm² may be adopted. Namely, in this method, the cell density at the start of proliferation culture (i.e., seeding density of the cells) can be, for example, 2001 to 1000000 cells/cm², and preferably 5000 to 20000 cells/cm². Needless to say, a seeding density lower than usual (0.002 to 2000 cells/cm²) may be employed in this method.

As described above, when the production culture and the recovery culture are alternately repeated to collect a supernatant of the production culture medium according to this method, the amounts of cytokines, extracellular matrix, and exosomes in the culture supernatant can increase as the number of times of the production culture increases. Therefore, in this method, the collection of a supernatant of the production culture medium is preferably performed after the performance of the second or subsequent production culture in the production culture medium. The collection of a supernatant of the production culture medium is more preferably performed after the third or subsequent production culture in the production culture medium is performed.

The collection of a supernatant of the production culture medium can be repeated, as long as the amounts of the cell products such as cytokines secreted by the stem cells are maintained. Namely, in this method, the number of repetitions of the production culture is not particularly limited. For example, the collection of a supernatant of the production culture medium can be performed after the performance of the second to tenth production culture in the production culture medium.

According to the "method of producing a culture supernatant of stem cells" described above, a culture supernatant of stem cells containing large amounts of cell products such as cytokines, extracellular matrix, and exosomes can be obtained.

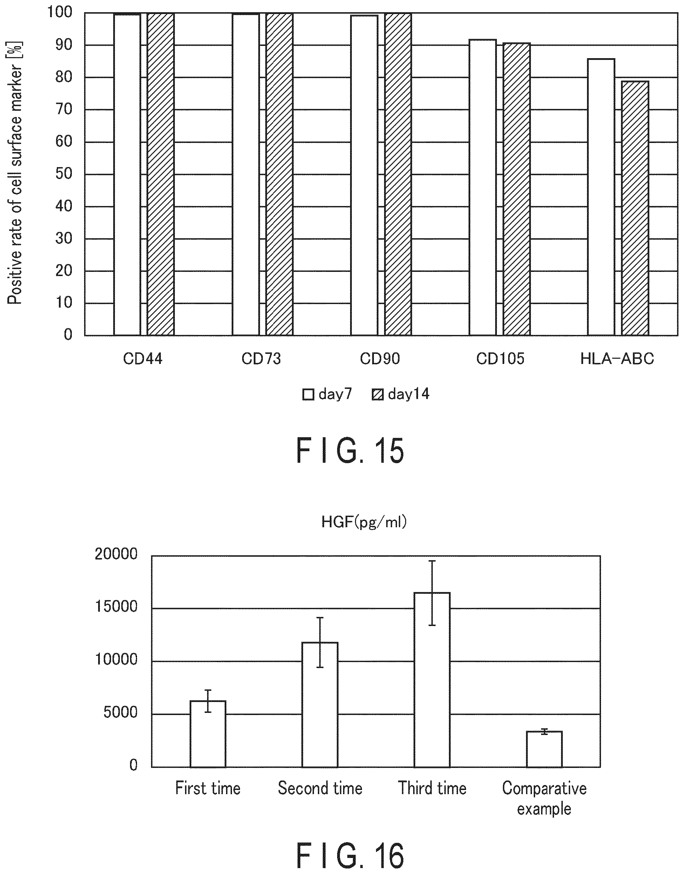

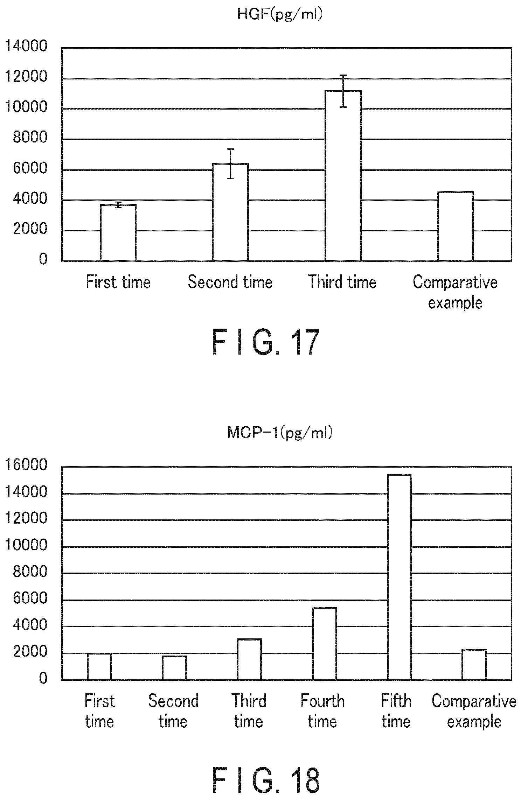

Specifically, according to the "method of producing a culture supernatant of stem cells" described above, a culture supernatant of stem cells containing 5000 pg/mL or more of HGF (hepatocyte growth factor) can be obtained (see FIGS. 16 and 17). Hereinafter, this culture supernatant will also be referred to as the culture supernatant according to the first embodiment. The culture supernatant contains HGF in an amount of, for example, 5000 pg/mL or more, preferably 10000 pg/mL or more, and more preferably 15000 pg/mL or more. The culture supernatant contains HGF in an amount of, for example, 5000 to 1000000 pg/mL, preferably 10000 to 1000000 pg/mL, and more preferably 15000 to 1000000 pg/mL. HGF is known to have angiogenesis effects and wound healing effects.

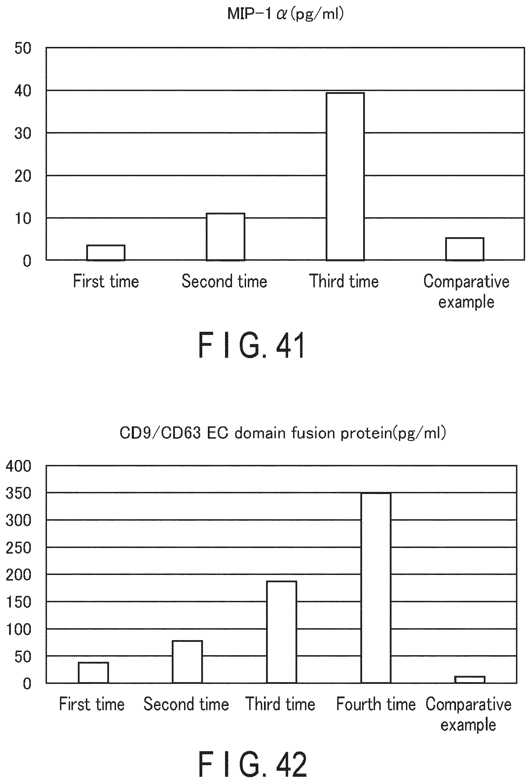

In addition, according to the "method of producing a culture supernatant of stem cells" described above, a culture supernatant of stem cells containing 50 pg/mL or more of CD9/CD63 EC domain fusion protein can be obtained (see FIG. 42). Hereinafter, this culture supernatant will also be referred to as the culture supernatant according to the second embodiment. The culture supernatant contains CD9/CD63 EC domain fusion protein in an amount of, for example, 50 pg/mL or more, preferably 100 pg/mL or more, and more preferably 200 pg/mL or more. The culture supernatant contains CD9/CD63 EC domain fusion protein in an amount of, for example, 50 to 100000 pg/mL, preferably 100 to 100000 pg/mL, and more preferably 200 to 100000 pg/mL. The CD9/CD63 EC domain fusion protein is a marker protein for exosomes, and a high content of CD9/CD63 EC domain fusion protein in the culture supernatant indicates a high content of exosomes in the culture supernatant. Exosomes are known to have angiogenesis effects and wound healing effects.

In a preferred embodiment, each of the culture supernatant according to the first embodiment and the culture supernatant according to the second embodiment can further contain 3000 pg/mL or more of MCP-1 (monocyte chemotactic protein-1), 1000 pg/mL or more of GRO (growth-related oncogene), and 5 µg/mL or more of fibronectin (see FIGS. 18 to 20). Each of the culture supernatant according to the first embodiment and the culture supernatant according to the second embodiment can further contain: MCP-1 in an amount of, for example, 3000 pg/mL or more, preferably 4000 pg/mL or more, and more preferably 6000 pg/mL or more; GRO in an amount of, for example, 1000 pg/mL or more, preferably 2000 pg/mL or more, and more preferably 4000 pg/mL or more; and fibronectin in an amount of, for example, 5 µg/mL or more, preferably 6 µg/mL or more, and more preferably 8 pg/mL or more. Each of the culture supernatant according to the first embodiment and the culture supernatant according to the second embodiment can further contain: MCP-1 in an amount of, for example, 3000 to 1000000 pg/mL, preferably 4000 to 1000000 pg/mL, and more preferably 6000 to 1000000 pg/mL; GRO in an amount of, for example, 1000 to 1000000 pg/mL, preferably 2000 to 1000000 pg/mL, and more preferably 4000 to 1000000 pg/mL; and fibronectin in an amount of, for example, 5 to 1000 pg/mL, preferably 6 to 1000 pg/mL, and more preferably 8 to 1000 µg/mL. All of MCP-1, GRO and fibronectin are known to have angiogenesis effects and wound healing effects. Since GRO is also known as CXCL1, it is also referred herein to as GRO/CXCL1.

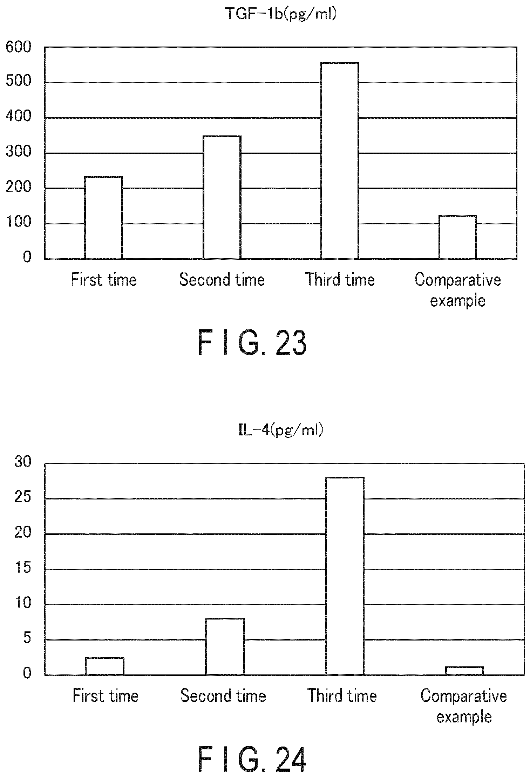

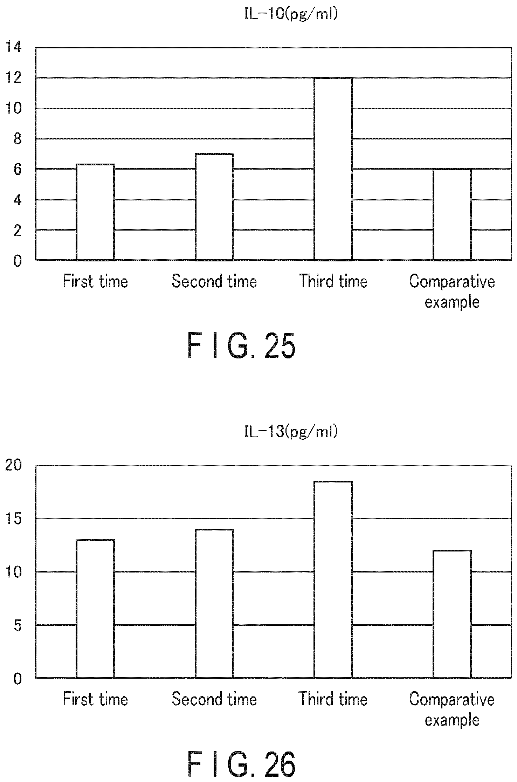

In a preferred embodiment, each of the culture supernatant according to the first embodiment and the culture supernatant according to the second embodiment can further contain 200 pg/mL or more of TGF-1b (transforming growth factor-lb), 5 pg/mL or more of IL-4 (interleukin-4), and 10 pg/mL or more of IL-10 (interleukin-10) (see FIGS. 23 to 25). Each of the culture supernatant according to the first embodiment and the culture supernatant according to the second embodiment can further contain: TGF-1b in an amount of, for example, 200 pg/mL or more, preferably 300 pg/mL or more, and more preferably 500 pg/mL or more; IL-4 in an amount of, for example, 5 pg/mL or more, preferably 10 pg/mL or more, and more preferably 20 pg/mL or more; and IL-10 in an amount of, for example, 8 pg/mL or more, preferably 10 pg/mL or more, and more preferably 12 pg/mL or more. Each of the culture supernatant according to the first embodiment and the culture supernatant according to the second embodiment can further contain: TGF-1b in an amount of, for example, 200 to 100000 pg/mL, preferably 300 to 100000 pg/mL, and more preferably 500 to 100000 pg/mL; IL-4 in an amount of, for example, 5 to 100000 pg/mL, preferably 10 to 100000 pg/mL, and more preferably 20 to 100000 pg/mL; and IL-10 in an amount of, for example, 8 to 100000 pg/mL, preferably 10 to 100000 pg/mL, and more preferably 12 to 100000 pg/mL. All of TGF-1b, IL-4, and IL-10 are known to have antiinflammatory effects.

Each of the culture supernatant according to the first embodiment and the culture supernatant according to the second embodiment is preferably free of at least one of insulin, transferrin, or albumin. It is more preferable that each of the culture supernatant according to the first embodiment and the culture supernatant according to the second embodiment does not contain any of insulin, transferrin, and albumin. In the production culture for obtaining a culture supernatant, a culture supernatant free of the above components can be obtained by using a culture medium free of the above components as the production culture medium. Considering that the culture supernatant is used as a therapeutic agent, it is preferable that the culture supernatant be free of the above components.

Also, each of the culture supernatant according to the first embodiment and the culture supernatant according to the second embodiment is preferably free of recombinant proteins. A recombinant protein-free culture supernatant can be obtained by using stem cells that have not been genetically engineered to produce recombinant proteins as stem cells. Considering that the culture supernatant is used as a therapeutic agent, it is preferable that the culture supernatant be free of recombinant proteins due to concerns over both the risk of secretion of an unexpected component into the culture supernatant due to genetic engineering of the cells and the occurrence of unknown side effects of the recombinant proteins produced.

In addition, each of the culture supernatant according to the first embodiment and the culture supernatant according to the second embodiment is preferably free of xenogeneic components. The term "xenogeneic" as used herein refers to a species different from the biological species of the stem cells used in order to obtain the culture supernatant. Considering that the culture supernatant is used as a therapeutic agent, it is preferable that the culture supernatant be free of xenogeneic components.

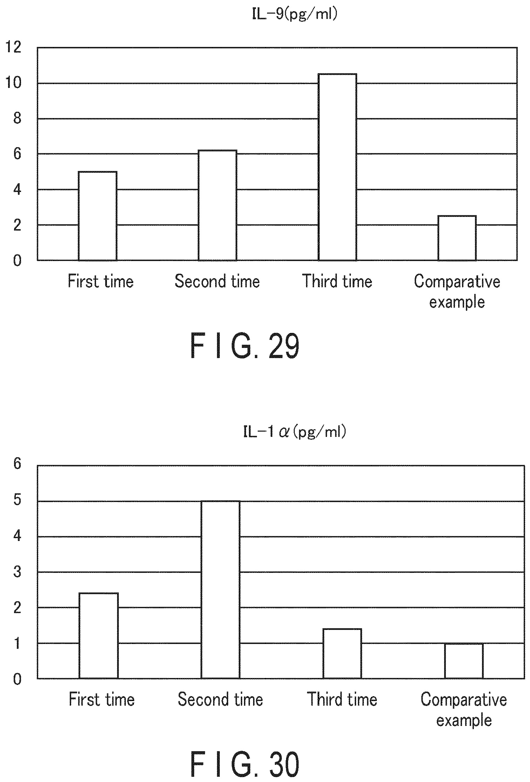

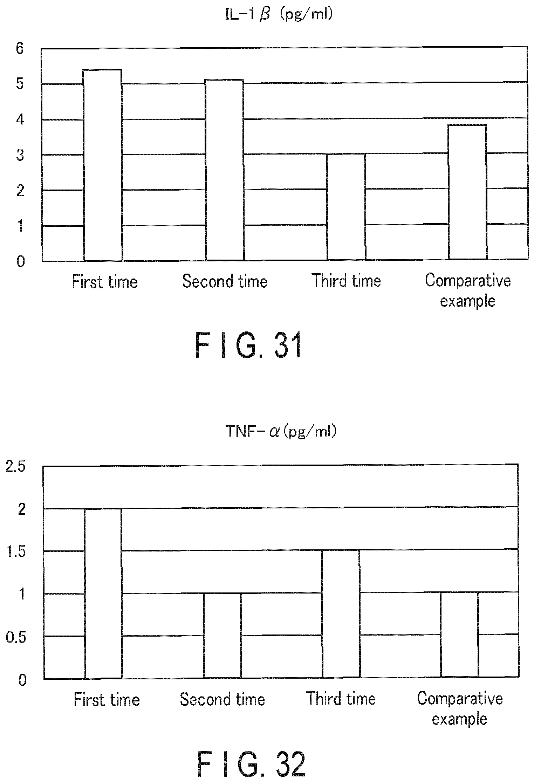

Each of the culture supernatant according to the first embodiment and the culture supernatant according to the second embodiment preferably contains at least one of IL-1α (interleukin-la), IL-1β (interleukin-1β), or TNF-α (tumor necrosis factor-a) in an amount of 0 to 15 pg/mL (see FIGS. 30 to 32). Each of the culture supernatant according to the first embodiment and the culture supernatant according to the second embodiment more preferably contains each of IL-1α, IL-1β, and TNF-α in an amount of 0 to 15 pg/mL. Since these cytokines are known as factors causing inflammatory symptoms, it is desirable that the culture supernatant does not contain them in large amounts.

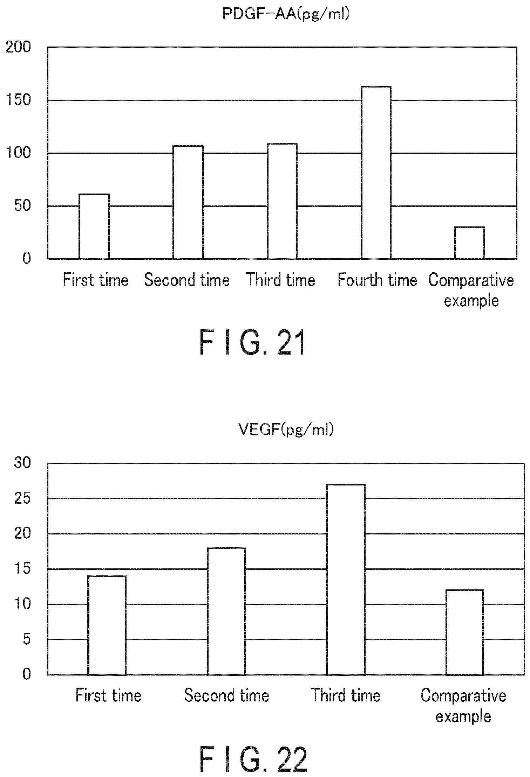

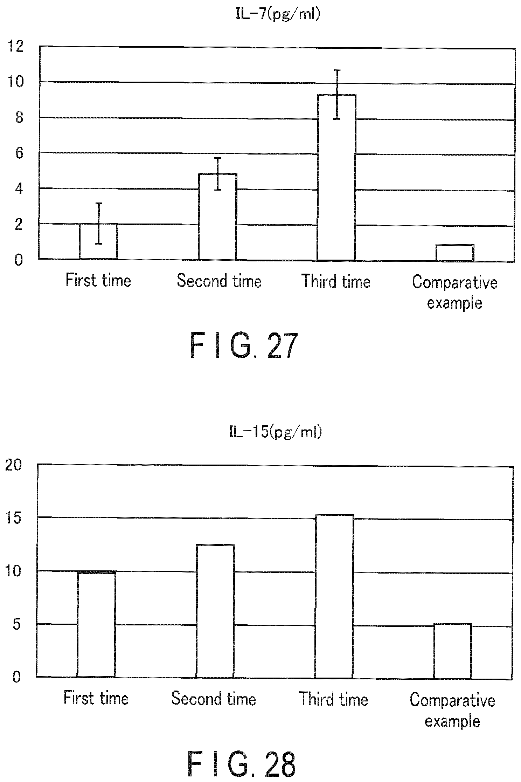

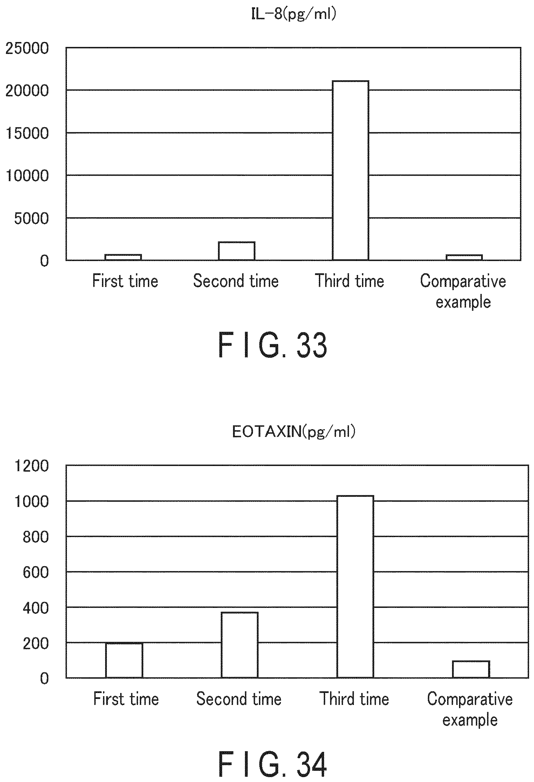

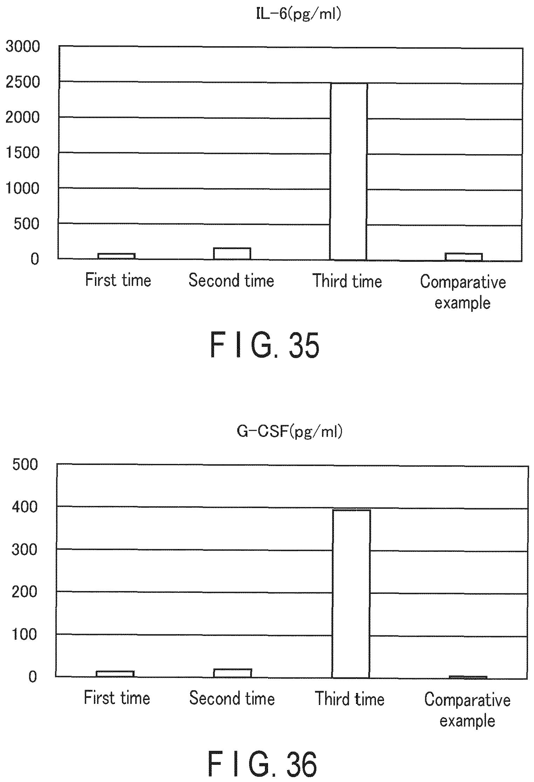

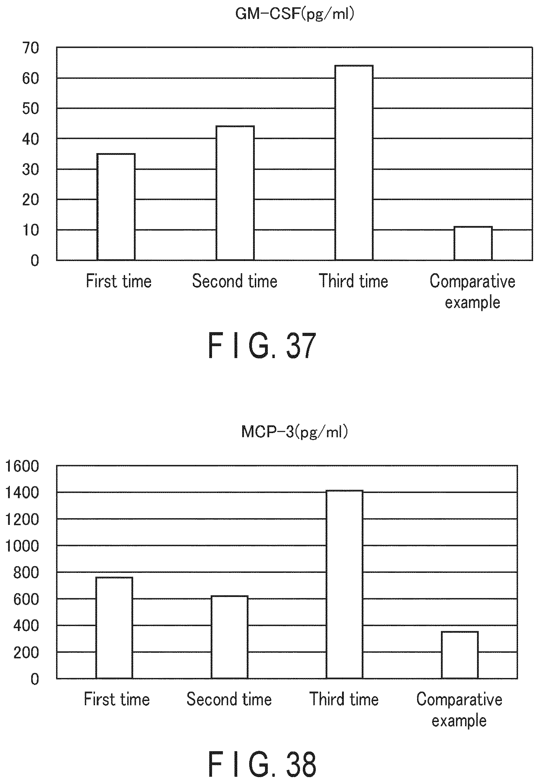

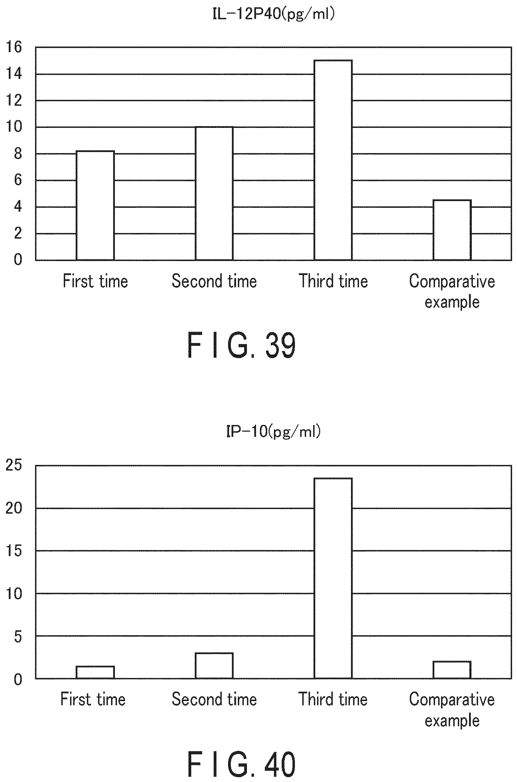

It is preferable that each of the culture supernatant according to the first embodiment and the culture supernatant according to the second embodiment contain 20 or more types of cytokines, and that each cytokine have a concentration of 10 pg/mL or more. The examples described later demonstrate that the culture supernatant collected after the third production culture contains, as cytokines, HGF, MCP-1, GRO, PDGF-AA (platelet-derived growth factor-AA), VEGF (vascular endothelial growth factor), TGF-1b, IL-4, IL-10, IL-13, IL-7, IL-15, IL-9, IL-8, EOTAXIN, IL-6, G-CSF (granulocyte-colony stimulating factor), GM-CSF (granulocyte macrophage-colony stimulating factor), MCP-3 (monocyte chemotactic protein-3), IL-12P40 (40 kDa subunit of interleukin-12), IP-10 (interferon gamma-induced protein-10), and MIP-1α (macrophage inflammatory protein-1α), and that each of these cytokines has a concentration of 10 pg/mL or more (See FIGS. 16 to 19, 21 to 29, and 33 to 41). The concentration of each of the 20 or more types of cytokines varies depending on the type of cytokine, but is, for example, 1000000 pg/mL or less.

Each of the culture supernatant according to the first embodiment and the culture supernatant according to the second embodiment is preferably a culture supernatant of mesenchymal stem cells. Each of the culture supernatant according to the first embodiment and the culture supernatant according to the second embodiment is more preferably a culture supernatant of umbilical cord-derived mesenchymal stem cells. A culture supernatant of mesenchymal stem cells can contain larger amounts of cytokines, extracellular matrix, and exosomes. A culture supernatant of umbilical cord-derived mesenchymal stem cells can contain particularly large amounts of cytokines, extracellular matrix, and exosomes.

The examples provided later describe a culture supernatant of mesenchymal stem cells; however, stem cells other than mesenchymal stem cells, such as pluripotent stem cells (e.g., ES cells and iPS cells) and somatic stem cells (e.g., neural stem cells, skin stem cells, liver stem cells, muscle stem cells, and adipose stem cells) are also known to secrete cytokines, extracellular matrix, and exosomes into a culture medium. Thus, even when the above-described "method of producing a culture supernatant of stem cells" is performed using stem cells other than mesenchymal stem cells, such as pluripotent stem cells (e.g., ES cells and iPS cells) and somatic stem cells (e.g., neural stem cells, skin stem cells, liver stem cells, muscle stem cells, and adipose stem cells), a culture supernatant containing large amounts of cytokines, extracellular matrix, and exosomes can be obtained as in the case of the "culture supernatant of mesenchymal stem cells".

Even when the "method of producing a culture supernatant of stem cells" is performed using cells, other than stem cells, that are known to secrete cytokines, extracellular matrix, and exosomes into a culture medium, a culture supernatant containing large amounts of cytokines, extracellular matrix, and exosomes can be obtained as in the case of the "culture supernatant of stem cells". Accordingly, the "method of producing a culture supernatant of stem cells" described above can be extended to "a method of producing a culture supernatant of cells", and the "culture supernatant of stem cells" described above can be extended to "a culture supernatant of cells".

### <6. Therapeutic Agent>

The inventors of the present application made a new discovery that when mesenchymal stem cells are cultured in a proliferation culture medium in the presence of a laminin fragment having integrin-binding activity according to the method described in <1. Method of Producing Proliferated Cells>, the obtained mesenchymal stem cells can significantly increase the blood flow in the ischemic lower limb of a lower-limb ischemia rat model (see Example 6 described later).

Thus, according to another aspect, there is provided a therapeutic agent containing the mesenchymal stem cell population of the present invention. As described above, the mesenchymal stem cell population of the present invention can be obtained by culturing mesenchymal stem cells in a proliferation culture medium in the presence of a laminin fragment having integrin-binding activity according to the method described in <1. Method of Producing Proliferated Cells>. As described above, the mesenchymal stem cell population of the present invention is characterized in that the positive rate of the specific cell surface marker, HLA-ABC is low, unlike the conventional knowledge regarding mesenchymal stem cell populations.

According to another aspect, there is provided a therapeutic agent containing the culture supernatant of the present invention. As described above, the culture supernatant of the present invention can be obtained by culturing mesenchymal stem cells in a proliferation culture medium in the presence of a laminin fragment having integrin-binding activity, followed by culturing the cells in a production culture medium and collecting a supernatant of the production culture medium, according to the method described in <2. Method of Producing Cell Product>. In addition, the culture supernatant of the present invention can be obtained by culturing mesenchymal stem cells in a proliferation culture medium in the presence of a laminin fragment having integrin-binding activity, followed by repeating the culturing performed in a production culture medium and the culturing performed in a recovery culture medium and collecting a supernatant of the production culture medium, according to the method described in <2. Method of Producing Cell Product>. As described above, the culture supernatant of the present invention is characterized in that it contains larger amounts of cytokines, extracellular matrix, and exosomes than conventional culture supernatants.

The mesenchymal stem cell population of the present invention has been demonstrated to secrete cytokines (e.g., HGF, MCP-1, GRO/CXCL1, PDGF-AA, VEGF, TGF-1b, IL-4, IL-10, IL-13, IL-7, IL-15, IL-9, IL-8, EOTAXIN, IL-6, G-CSF, GM-CSF, MCP-3, IL-12P40, IP-10, and MIP-1α), extracellular matrix (e.g., fibronectin), and exosomes. The culture supernatant of the present invention has been demonstrated to contain cytokines, extracellular matrix, and exosomes, secreted by mesenchymal stem cells. Therefore, a therapeutic agent containing the mesenchymal stem cell population of the present invention or the culture supernatant of the present invention can be used to treat diseases on which cytokines are known to have therapeutic effects, diseases on which extracellular matrices are known to have therapeutic effects, and diseases on which exosomes are known to have therapeutic effects, in addition to diseases on which mesenchymal stem cells are known to have therapeutic effects and diseases on which a culture supernatant of mesenchymal stem cells are known to have therapeutic effects.

For example, a therapeutic agent containing the mesenchymal stem cell population of the present invention or the culture supernatant of the present invention can be used for the treatment of: ischemic diseases such as lower-limb ischemia, myocardial infarction, cerebral infarction, spinal cord infarction, and chronic arterial occlusive disease; wounds such as an epithelial wound and thermal burn; and sarcopenia due to aging, since HGF, MCP-1, MCP-3, GRO/CXCL1, fibronectin, PDGF-AA, VEGF, IL-8, EOTAXIN, IL-6, IP-10, and exosomes are known to have angiogenesis effects.

A therapeutic agent containing the mesenchymal stem cell population of the present invention or the culture supernatant of the present invention can also be used for the treatment of: arthritides such as rheumatoid arthritis, spinal disc herniation, and osteoarthritis; inflammatory diseases such as nephritis, keratitis, and cytokine storm; and mental disorders such as autism and insomnia that are expected to stem from neuroinflammation as one cause, since TGF-1b, IL-4, IL-10, and IL-13 are known as cytokines having anti-inflammatory effects.

A therapeutic agent containing the mesenchymal stem cell population of the present invention or the culture supernatant of the present invention can also be used for the treatment of: immunologic diseases such as GVHD (graft versus host disease), Sjögren's syndrome, atopic dermatitis, connective tissue disease, multiple sclerosis, and autoimmune disease; and cancer diseases, since IL-7, IL-15, GM-CSF, and G-CSF are considered to be involved in immunomodulation.

When the mesenchymal stem cell population is used as an active ingredient of a therapeutic agent, long-term therapeutic effects can be expected since the mesenchymal stem cells of the present invention can continuously secrete cell products such as cytokines. On the other hand, when the culture supernatant of mesenchymal stem cells is used as an active ingredient of a therapeutic agent, sufficient therapeutic effects can be expected since the culture supernatant of the present invention contains large amounts of cell products such as cytokines.

A therapeutic agent containing the mesenchymal stem cell population of the present invention and a therapeutic agent containing the culture supernatant of the present invention (hereinafter, collectively referred to as therapeutic agents) are, for example, liquid preparations, preferably injectable liquid preparations or external liquid preparations. The therapeutic agents may be diluted with a pharmaceutically acceptable medium. The pharmaceutically acceptable medium is, for example, a culture medium of mesenchymal stem cells or an infusional preparation. The therapeutic agents may contain additives for increasing storage stability, isotonicity, absorbability, and/or viscosity. Alternatively, the therapeutic agents may be in the form of a cell sheet when they contain the mesenchymal stem cell population.

The dose of the therapeutic agents can be appropriately determined depending on the target disease, age, body weight, symptoms, and the like. When a therapeutic agent contains the mesenchymal stem cell population, a single dose of the therapeutic agent is, for example, 1000 to 100000000 cells/kg, preferably 100000 to 10000000 cells/kg. This dose of the therapeutic agent as a single dose may be administered multiple times, or this dose of the therapeutic agent may be divided into multiple doses. When a therapeutic agent contains the culture supernatant, a single dose of the therapeutic agent is, for example, 0.01 to 100 mL/kg, preferably 0.1 to 10 mL/kg, in terms of the amount (mL) of the culture supernatant before dilution. This dose of the therapeutic agent as a single dose may be administered multiple times, or this dose of the therapeutic agent may be divided into multiple doses.

A method of administering the therapeutic agents is not particularly limited. Examples thereof include intravenous injection, intra-arterial injection, subcutaneous injection, intra-lymph node injection, intraperitoneal injection, application of a liquid preparation to a tissue, attachment of a cell sheet, direct injection or direct transplantation to a local site, and the like.

### <7. Preferred Embodiments>

Hereinafter, the preferred embodiments of the present invention will be described.

### <7-1. Mesenchymal Stem Cell Population and Method of Producing Same>

[C1] A method of producing a mesenchymal stem cell population having a reduced HLA-ABC positive rate, the method including: culturing mesenchymal stem cells in a proliferation culture medium in a presence of a culture substrate selected from a laminin fragment having integrin-binding activity and a modified form thereof, thereby obtaining proliferated cells.

[C2] The method according to [C1], wherein the mesenchymal stem cells are umbilical cord-derived mesenchymal stem cells, bone marrow-derived mesenchymal stem cells, adipose-derived mesenchymal stem cells, placenta-derived mesenchymal stem cells, or umbilical cord blood-derived mesenchymal stem cells.

[C3] The method according to [C1] or [C2], wherein the mesenchymal stem cells are umbilical cord-derived mesenchymal stem cells or adipose-derived mesenchymal stem cells, preferably umbilical cord-derived mesenchymal stem cells.

[C4] The method according to [C1], wherein the mesenchymal stem cells are human mesenchymal stem cells.

[C5] The method according to [C1] or [C2], wherein the mesenchymal stem cells are human umbilical cord-derived mesenchymal stem cells, human bone marrow-derived mesenchymal stem cells, human adipose-derived mesenchymal stem cells, human placenta-derived mesenchymal stem cells, or human umbilical cord blood-derived mesenchymal stem cells.

[C6] The method according to any one of [C1] to [C3], wherein the mesenchymal stem cells are human umbilical cord-derived mesenchymal stem cells or human adipose-derived mesenchymal stem cells, preferably human umbilical cord-derived mesenchymal stem cells.

[C7] The method according to any one of [C1] to [C6], wherein the proliferation culture medium is a proliferation culture medium containing a protein that promotes proliferation of the stem cells.

[C8] The method according to any one of [C1] to [C7], wherein the proliferation culture medium is a culture medium containing a basal medium for cell culture supplemented with a growth factor.

[C9] The method according to any one of [C1] to [C8], wherein the proliferation culture medium is a basal medium for cell culture supplemented with a growth factor.

[C10] The method according to any one of [C1] to [C9], wherein the culturing is performed until the cells reach a confluent state.

[C11] The method according to any one of [C1] to [C10], wherein the culturing is performed in a culture vessel having a bottom area of 500 cm² or more.

[C12] The method according to any one of [C1] to [C11], wherein the culturing is performed in a culture vessel having a bottom area of 500 to 10000 cm².

[C13] The method according to any one of [C1] to [C12], wherein the culture substrate is a laminin fragment having integrin-binding activity.

[C14] The method according to [C13], wherein the laminin fragment is a human-derived laminin fragment.

[C15] The method according to [C13] or [C14], wherein the laminin fragment is a laminin E8 fragment.

[C16] The method according to any one of [C13] to [C15], wherein the laminin fragment is a laminin 511 E8 fragment, a laminin 521 E8 fragment, a laminin 411 E8 fragment, a laminin 421 E8 fragment, a laminin 332 E8 fragment, a laminin 311 E8 fragment, a laminin 321 E8 fragment, a laminin 211 E8 fragment, a laminin 221 E8 fragment, a laminin 213 E8 fragment, a laminin 111 E8 fragment, or a laminin 121 E8 fragment.

[C17] The method according to any one of [C13] to [C16], wherein the laminin fragment is a laminin 511 E8 fragment.

[C18] The method according to any one of [C1] to [C12], wherein the culture substrate is a modified form of a laminin fragment having integrin-binding activity.

[C19] The method according to [C18], wherein the modified form is a complex of a laminin fragment having integrin-binding activity and another functional molecule.

[C20] The method according to [C18] or [C19], wherein the modified form is a complex of a laminin fragment having integrin-binding activity and a growth factor-binding molecule.

[C21] The method according to [C19] or [C20], wherein the laminin fragment is a laminin E8 fragment.

[C22] The method according to [C21], wherein the laminin E8 fragment is a laminin 511 E8 fragment, a laminin 521 E8 fragment, a laminin 411 E8 fragment, a laminin 421 E8 fragment, a laminin 332 E8 fragment, a laminin 311 E8 fragment, a laminin 321 E8 fragment, a laminin 211 E8 fragment, a laminin 221 E8 fragment, a laminin 213 E8 fragment, a laminin 111 E8 fragment, or a laminin 121 E8 fragment.

[C23] The method according to [C21] or [C22], wherein the laminin E8 fragment is a laminin 511 E8 fragment.

[C24] The method according to [C21] or [C22], wherein the laminin E8 fragment is a laminin 421 E8 fragment.

[C25] The method according to any one of [C20] to [C24], wherein the growth factor-binding molecule is heparan sulfate.

[C26] The method according to any one of [C18] to [C25], wherein the modified form is a complex of a laminin 511 E8 fragment and heparan sulfate.

[C27] The method according to any one of [C18] to [C25], wherein the modified form is a complex of a laminin 421 E8 fragment and heparan sulfate.

[C28] The method according to any one of [C1] to [C27], wherein a concentration of the laminin fragment or modified form thereof in the proliferation culture medium is 0.005 µg to 2 µg per 1 cm² culture area of the culture vessel.

[C29] The method according to any one of [C1] to [C28], wherein a concentration of the laminin fragment or modified form thereof in the proliferation culture medium is 0.01 µg to 0.5 µg per 1 cm² culture area of the culture vessel.

[C30] The method according to any one of [C1] to [C29], wherein a concentration of the laminin fragment or modified form thereof in the proliferation culture medium is 0.05 µg to 0.25 µg per 1 cm² culture area of the culture vessel.

[C31] The method according to any one of [C1] to [C30], wherein the reduced HLA-ABC positive rate is 70% or less, preferably 60% or less, more preferably 50% or less, still more preferably 40% or less, still more preferably 30% or less, still more preferably 20% or less, and still more preferably 10% or less.

[D1] A mesenchymal stem cell population, including HLA-ABC positive mesenchymal stem cells at a ratio of 70% or less.

[D2] The mesenchymal stem cell population according to [D1], wherein the ratio of the HLA-ABC positive mesenchymal stem cells is 60% or less, preferably 50% or less, more preferably 40% or less, still more preferably 30% or less, still more preferably 20% or less, and still more preferably 10% or less.

[D3] The mesenchymal stem cell population according to [D1] or [D2], wherein the mesenchymal stem cell population includes CD105 positive mesenchymal stem cells at a ratio of 50% or less.

[D4] The mesenchymal stem cell population according to [D3], wherein the ratio of the CD105 positive mesenchymal stem cells is 40% or less, preferably 30% or less, more preferably 20% or less, and still more preferably 10% or less.

[D5] A mesenchymal stem cell population obtainable by the method according to any one of [C1] to [C31].

[D6] The mesenchymal stem cell population according to any one of [D1] to [D4], wherein the mesenchymal stem cell population is obtainable by the method according to any one of [C1] to [C31].

### <7-2. Culture Supernatant of Stem Cells and Method of Producing Same>

[E1] A method of producing a culture supernatant of stem cells, the method including:
culturing stem cells through adhesion culture in a proliferation culture medium in a presence of a culture substrate selected from a laminin fragment having integrin-binding activity and a modified form thereof, thereby obtaining proliferated cells in an adhered state;
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells, and the method further includes collecting a supernatant of the production culture medium after performing the culturing in the production culture medium.

[E2] The method according to [E1], wherein the collecting of the supernatant of the production culture medium is performed, after the culturing in the production culture medium is performed for a second or subsequent time, preferably after the culturing in the production culture medium is performed for a third or subsequent time.

[E3] The method according to [E1] or [E2], wherein the stem cells are somatic stem cells such as mesenchymal stem cells, neural stem cells, skin stem cells, liver stem cells, muscle stem cells, or adipose stem cells; or pluripotent stem cells such as induced pluripotent stem cells (iPS cells) or embryonic stem cells (ES cells).

[E4] The method according to any one of [E1] to [E3], wherein the stem cells are mesenchymal stem cells.

[E5] The method according to any one of [E1] to [E4], wherein the stem cells are umbilical cord-derived mesenchymal stem cells, bone marrow-derived mesenchymal stem cells, adipose-derived mesenchymal stem cells, placenta-derived mesenchymal stem cells, or umbilical cord blood-derived mesenchymal stem cells.

[E6] The method according to any one of [E1] to [E5], wherein the stem cells are umbilical cord-derived mesenchymal stem cells or adipose-derived mesenchymal stem cells, preferably umbilical cord-derived mesenchymal stem cells.

[E7] The method according to [E1] or [E2], wherein the stem cells are human stem cells.

[E8] The method according to any one of [E1] to [E3], wherein the stem cells are human somatic stem cells such as human mesenchymal stem cells, human neural stem cells, human skin stem cells, human liver stem cells, human muscle stem cells, or human adipose stem cells; or human pluripotent stem cells such as human induced pluripotent stem cells (human iPS cells) or human embryonic stem cells (human ES cells).

[E9] The method according to any one of [E1] to [E4], wherein the stem cells are human mesenchymal stem cells.

[E10] The method according to any one of [E1] to [E5], wherein the stem cells are human umbilical cord-derived mesenchymal stem cells, human bone marrow-derived mesenchymal stem cells, human adipose-derived mesenchymal stem cells, human placenta-derived mesenchymal stem cells, or human umbilical cord blood-derived mesenchymal stem cells.

[E11] The method according to any one of [E1] to [E6], wherein the stem cells are human umbilical cord-derived mesenchymal stem cells or human adipose-derived mesenchymal stem cells, preferably human umbilical cord-derived mesenchymal stem cells.

[E12] The method according to any one of [E1] to [E11], wherein the proliferation culture medium is a proliferation culture medium containing a protein that promotes proliferation of the stem cells.

[E13] The method according to any one of [E1] to [E12], wherein the proliferation culture medium is a culture medium containing a basal medium for cell culture supplemented with a growth factor.

[E14] The method according to any one of [E1] to [E13], wherein the proliferation culture medium is a basal medium for cell culture supplemented with a growth factor.

[E15] The method according to any one of [E1] to [E14], wherein the culturing in the proliferation culture medium is performed until the cells reach a confluent state.

[E16] The method according to any one of [E1] to [E15], wherein the culturing in the proliferation culture medium is performed in a culture vessel having a bottom area of 500 cm² or more.

[E17] The method according to any one of [E1] to [E16], wherein the culturing in the proliferation culture medium is performed in a culture vessel having a bottom area of 500 to 10000 cm².

[E18] The method according to any one of [E1] to [E17], wherein the culture substrate is a laminin fragment having integrin-binding activity.

[E19] The method according to [E18], wherein the laminin fragment is a human-derived laminin fragment.

[E20] The method according to [E18] or [E19], wherein the laminin fragment is a laminin E8 fragment.

[E21] The method according to any one of [E18] to [E20], wherein the laminin fragment is a laminin 511 E8 fragment, a laminin 521 E8 fragment, a laminin 411 E8 fragment, a laminin 421 E8 fragment, a laminin 332 E8 fragment, a laminin 311 E8 fragment, a laminin 321 E8 fragment, a laminin 211 E8 fragment, a laminin 221 E8 fragment, a laminin 213 E8 fragment, a laminin 111 E8 fragment, or a laminin 121 E8 fragment.

[E22] The method according to any one of [E18] to [E21], wherein the laminin fragment is a laminin 511 E8 fragment.

[E23] The method according to any one of [E1] to [E17], wherein the culture substrate is a modified form of a laminin fragment having integrin-binding activity.

[E24] The method according to [E23], wherein the modified form is a complex of a laminin fragment having integrin-binding activity and another functional molecule.

[E25] The method according to [E23] or [E24], wherein the modified form is a complex of a laminin fragment having integrin-binding activity and a growth factor-binding molecule.

[E26] The method according to [E24] or [E25], wherein the laminin fragment is a laminin E8 fragment.

[E27] The method according to [E26], wherein the laminin E8 fragment is a laminin 511 E8 fragment, a laminin 521 E8 fragment, a laminin 411 E8 fragment, a laminin 421 E8 fragment, a laminin 332 E8 fragment, a laminin 311 E8 fragment, a laminin 321 E8 fragment, a laminin 211 E8 fragment, a laminin 221 E8 fragment, a laminin 213 E8 fragment, a laminin 111 E8 fragment, or a laminin 121 E8 fragment.

[E28] The method according to [E26] or [E27], wherein the laminin E8 fragment is a laminin 511 E8 fragment.

[E29] The method according to [E26] or [E27], wherein the laminin E8 fragment is a laminin 421 E8 fragment.

[E30] The method according to any one of [E25] to [E29], wherein the growth factor-binding molecule is heparan sulfate.

[E31] The method according to any one of [E23] to [E30], wherein the modified form is a complex of a laminin 511 E8 fragment and heparan sulfate.

[E32] The method according to any one of [E23] to [E30], wherein the modified form is a complex of a laminin 421 E8 fragment and heparan sulfate.

[E33] The method according to any one of [E1] to [E32], wherein a concentration of the laminin fragment or modified form thereof in the proliferation culture medium is 0.005 µg to 2 µg per 1 cm² culture area of the culture vessel.

[E34] The method according to any one of [E1] to [E33], wherein a concentration of the laminin fragment or modified form thereof in the proliferation culture medium is 0.01 µg to 0.5 µg per 1 cm² culture area of the culture vessel.

[E35] The method according to any one of [E1] to [E34], wherein a concentration of the laminin fragment or modified form thereof in the proliferation culture medium is 0.05 µg to 0.25 µg per 1 cm² culture area of the culture vessel.

[E36] The method according to any one of [E1] to [E35], wherein the production culture medium is a culture medium free of a xenogeneic component.

[E37] The method according to any one of [E1] to [E36], wherein the production culture medium is a culture medium free of a cytokine or insulin.

[E38] The method according to any one of [E1] to [E37], wherein the production culture medium is a protein-free culture medium.

[E39] The method according to any one of [E1] to [E38], wherein the production culture medium is a serum-free culture medium.

[E40] The method according to any one of [E1] to [E39], wherein the production culture medium is a culture medium containing a basal medium for cell culture, or a culture medium containing a basal medium for cell culture supplemented with a nutrient component for the cells.

[E41] The method according to any one of [E1] to [E40], wherein the production culture medium is a basal medium for cell culture, or a basal medium for cell culture supplemented with a nutrient component for the cells.

[E42] The method according to any one of [E1] to [E41], wherein the production culture medium is a basal medium for cell culture supplemented with a nutrient component for the cells, preferably an amino acid.

[E43] The method according to any one of [E1] to [E42], wherein the production culture medium is free of a laminin fragment or a modified form thereof.

[E44] The method according to any one of [E1] to [E43], wherein the culturing in the production culture medium is performed for 0.5 to 10 days, preferably 2 to 5 days.

[E45] The method according to any one of [E1] to [E44], wherein the recovery culture medium is a proliferation culture medium of the stem cells.

[E46] The method according to any one of [E1] to [E45], wherein the recovery culture medium is a culture medium containing a protein that promotes proliferation of the stem cells.

[E47] The method according to any one of [E1] to [E46], wherein the recovery culture medium is a culture medium containing a basal medium for cell culture supplemented with a growth factor.

[E48] The method according to any one of [E1] to [E47], wherein the recovery culture medium is a basal medium for cell culture supplemented with a growth factor.

[E49] The method according to any one of [E1] to [E48], wherein the recovery culture medium has a composition same as a composition of the proliferation culture medium.

[E50] The method according to any one of [E1] to [E49], wherein the recovery culture medium is free of a laminin fragment or a modified form thereof.

[E51] The method according to any one of [E1] to [E50], wherein the culturing in the recovery culture medium is performed for 0.5 to 10 days, preferably 2 to 5 days.

[E52] The method according to any one of [E1] to [E51], wherein a cycle of the culturing performed in the production culture medium and the culturing performed in the recovery culture medium is repeated 2 to 10 times.

[F1] A culture supernatant of stem cells, the culture supernatant containing 5000 pg/mL or more of HGF.

[F2] The culture supernatant according to [F1], wherein the culture supernatant contains HGF in an amount of 10000 pg/mL or more, preferably 15000 pg/mL or more.

[F3] The culture supernatant according to [F1] or [F2], wherein the culture supernatant contains HGF in an amount of 5000 to 1000000 pg/mL, preferably 10000 to 1000000 pg/mL, and more preferably 15000 to 1000000 pg/mL.

[F4] A culture supernatant of stem cells, the culture supernatant containing 50 pg/mL or more of CD9/CD63 EC domain fusion protein.

[F5] The culture supernatant according to [F4], wherein the culture supernatant contains CD9/CD63 EC domain fusion protein in an amount of 100 pg/mL or more, preferably 200 pg/mL or more.

[F6] The culture supernatant according to [F4] or [F5], wherein the culture supernatant contains CD9/CD63 EC domain fusion protein in an amount of 50 to 100000 pg/mL, preferably 100 to 100000 pg/mL, and more preferably 200 to 100000 pg/mL.

[F7] The culture supernatant according to any one of [F1] to [F3], wherein the culture supernatant further contains 50 pg/mL or more of CD9/CD63 EC domain fusion protein.

[F8] The culture supernatant according to any one of [F1] to [F3], wherein the culture supernatant further contains CD9/CD63 EC domain fusion protein in an amount of 100 pg/mL or more, preferably 200 pg/mL or more.

[F9] The culture supernatant according to any one of [F1] to [F3], wherein the culture supernatant further contains CD9/CD63 EC domain fusion protein in an amount of 50 to 100000 pg/mL, preferably 100 to 100000 pg/mL, and more preferably 200 to 100000 pg/mL.

[F10] The culture supernatant according to any one of [F1] to [F9], wherein the culture supernatant further contains 3000 pg/mL or more of MCP-1, 1000 pg/mL or more of GRO, and 5 µg/mL or more of fibronectin.

[F11] The culture supernatant according to any one of [F1] to [F9], wherein the culture supernatant further contains: MCP-1 in an amount of 4000 pg/mL or more, preferably 6000 pg/mL or more; GRO in an amount of 2000 pg/mL or more, preferably 4000 pg/mL or more; and fibronectin in an amount of 6 pg/mL or more, preferably 8 µg/mL or more.

[F12] The culture supernatant according to any one of [F1] to [F9], wherein the culture supernatant further contains: MCP-1 in an amount of 3000 to 1000000 pg/mL, preferably 4000 to 1000000 pg/mL, and more preferably 6000 to 1000000 pg/mL; GRO in an amount of 1000 to 1000000 pg/mL, preferably 2000 to 1000000 pg/mL, and more preferably 4000 to 1000000 pg/mL; and fibronectin in an amount of 5 to 1000 µg/mL, preferably 6 to 1000 pg/mL, and more preferably 8 to 1000 µg/mL.

[F13] The culture supernatant according to any one of [F1] to [F12], wherein the culture supernatant further contains 200 pg/mL or more of TGF-1b, 5 pg/mL or more of IL-4, and 8 pg/mL or more of IL-10.

[F14] The culture supernatant according to any one of [F1] to [F12], wherein the culture supernatant further contains: TGF-1b in an amount of 300 pg/mL or more, preferably 500 pg/mL or more; IL-4 in an amount of 10 pg/mL or more, preferably 20 pg/mL or more; and IL-10 in an amount of 10 pg/mL or more, preferably 12 pg/mL or more.

[F15] The culture supernatant according to any one of [F1] to [F12], wherein the culture supernatant further contains: TGF-1b in an amount of 200 to 100000 pg/mL, preferably 300 to 100000 pg/mL, and more preferably 500 to 100000 pg/mL; IL-4 in an amount of 5 to 100000 pg/mL, preferably 10 to 100000 pg/mL, and more preferably 20 to 100000 pg/mL; and IL-10 in an amount of 8 to 100000 pg/mL, preferably 10 to 100000 pg/mL, and more preferably 12 to 100000 pg/mL.

[F16] The culture supernatant according to any one of [F1] to [F15], wherein the culture supernatant is free of at least one of insulin, transferrin, or albumin.

[F17] The culture supernatant according to any one of [F1] to [F16], wherein the culture supernatant is free of all of insulin, transferrin, and albumin.

[F18] The culture supernatant according to any one of [F1] to [F17], wherein the culture supernatant is free of a recombinant protein.

[F19] The culture supernatant according to any one of [F1] to [F18], wherein the culture supernatant contains at least one of IL-1α, IL-1β, or TNF-α in an amount of 0 to 15 pg/mL.

[F20] The culture supernatant according to any one of [F1] to [F19], wherein the culture supernatant contains each of IL-1α, IL-1β, and TNF-α in an amount of 0 to 15 pg/mL.

[F21] The culture supernatant according to any one of [F1] to [F20], wherein the culture supernatant contains 20 or more types of cytokines, and each cytokine has a concentration of 10 pg/mL or more.

[F22] The culture supernatant according to any one of [F1] to [F21], wherein the culture supernatant is a culture supernatant of somatic stem cells such as mesenchymal stem cells, neural stem cells, skin stem cells, liver stem cells, muscle stem cells, or adipose stem cells; or a culture supernatant of pluripotent stem cells such as induced pluripotent stem cells (iPS cells) or embryonic stem cells (ES cells).

[F23] The culture supernatant according to any one of [F1] to [F22], wherein the culture supernatant is a culture supernatant of mesenchymal stem cells.

[F24] The culture supernatant according to any one of [F1] to [F23], wherein the culture supernatant is a culture supernatant of umbilical cord-derived mesenchymal stem cells, a culture supernatant of bone marrow-derived mesenchymal stem cells, a culture supernatant of adipose-derived mesenchymal stem cells, a culture supernatant of placenta-derived mesenchymal stem cells, or a culture supernatant of umbilical cord blood-derived mesenchymal stem cells.

[F25] The culture supernatant according to any one of [F1] to [F24], wherein the culture supernatant is a culture supernatant of umbilical cord-derived mesenchymal stem cells or a culture supernatant of adipose-derived mesenchymal stem cells, preferably a culture supernatant of umbilical cord-derived mesenchymal stem cells.

[F26] The culture supernatant according to any one of [F1] to [F23], wherein the culture supernatant is a culture supernatant of human mesenchymal stem cells.

[F27] The culture supernatant according to any one of [F1] to [F24], wherein the culture supernatant is a culture supernatant of human umbilical cord-derived mesenchymal stem cells, a culture supernatant of human bone marrow-derived mesenchymal stem cells, a culture supernatant of human adipose-derived mesenchymal stem cells, a culture supernatant of human placenta-derived mesenchymal stem cells, or a culture supernatant of human umbilical cord blood-derived mesenchymal stem cells.

[F28] The culture supernatant according to any one of [F1] to [F25], wherein the culture supernatant is a culture supernatant of human umbilical cord-derived mesenchymal stem cells or a culture supernatant of human adipose-derived mesenchymal stem cells, preferably a culture supernatant of human umbilical cord-derived mesenchymal stem cells.

[F29] A culture supernatant obtainable by the method according to any one of [E1] to [E52].

[F30] The culture supernatant according to any one of [F1] to [F28], wherein the culture supernatant is obtainable by the method according to any one of [E1] to [E52] .

### <7-3. Therapeutic Agent>

[G1] A therapeutic agent containing the mesenchymal stem cell population according to any one of [D1] to [D6] or the culture supernatant according to any one of [F1] to [F30].

[G2] A therapeutic agent containing the mesenchymal stem cell population according to any one of [D1] to [D6].

[G3] A therapeutic agent containing the culture supernatant according to any one of [F1] to [F30].

[G4] The therapeutic agent according to any one of [G1] to [G3], wherein the therapeutic agent is for treatment of: an ischemic disease such as lower-limb ischemia, myocardial infarction, cerebral infarction, spinal cord infarction, or chronic arterial occlusive disease; a wound such as an epithelial wound or thermal burn; sarcopenia due to aging; an arthritis such as rheumatoid arthritis, spinal disc herniation, or osteoarthritis; an inflammatory disease such as nephritis, keratitis, or cytokine storm; a mental disorder such as autism or insomnia which is expected to stem from neuroinflammation as one cause; an immunologic disease such as GVHD (graft versus host disease), Sjögren's syndrome, atopic dermatitis, connective tissue disease, multiple sclerosis, or autoimmune disease; or a cancer disease.

[G5] The therapeutic agent according to any one of [G1] to [G4], wherein the therapeutic agent is for treatment of an ischemic disease; preferably lower-limb ischemia, myocardial infarction, cerebral infarction, spinal cord infarction, or chronic arterial occlusive disease; and more preferably lower-limb ischemia.

[G6] A method of treating an injury and disease, the method including administering the mesenchymal stem cell population according to any one of [D1] to [D6] or the culture supernatant according to any one of [F1] to [F30] to a subject.

[G7] A method of treating an injury and disease, the method including administering the mesenchymal stem cell population according to any one of [D1] to [D6] to a subject.

[G8] A method of treating an injury and disease, the method including administering the culture supernatant according to any one of [F1] to [F30] to a subject.

[G9] The method according to any one of [G6] to [G8], wherein the injury and disease are: an ischemic disease such as lower-limb ischemia, myocardial infarction, cerebral infarction, spinal cord infarction, or chronic arterial occlusive disease; a wound such as an epithelial wound or thermal burn; sarcopenia due to aging; an arthritis such as rheumatoid arthritis, spinal disc herniation, or osteoarthritis; an inflammatory disease such as cytokine storm, nephritis, or keratitis; a mental disorder such as autism or insomnia which is expected to stem from neuroinflammation as one cause; an immunologic disease such as GVHD (graft versus host disease), Sjögren's syndrome, atopic dermatitis, connective tissue disease, multiple sclerosis, or autoimmune disease; or a cancer disease.

[G10] The method according to any one of [G6] to [G9], wherein the injury and disease are ischemic disease; preferably lower-limb ischemia, myocardial infarction, cerebral infarction, spinal cord infarction, or chronic arterial occlusive disease; and more preferably lower-limb ischemia.

[G11] The method according to any one of [G6] to [G10], wherein the subject is a mammal, preferably a human.

[G12] Use of the mesenchymal stem cell population according to any one of [D1] to [D6] or the culture supernatant according to any one of [F1] to [F30] for the manufacture of a therapeutic agent.

[G13] Use of the mesenchymal stem cell population according to any one of [D1] to [D6] for the manufacture of a therapeutic agent.

[G14] Use of the culture supernatant according to any one of [F1] to [F30] for the manufacture of a therapeutic agent.

[G15] The use according to any one of [G12] to [G14], wherein the therapeutic agent is for treatment of: an ischemic disease such as lower-limb ischemia, myocardial infarction, cerebral infarction, spinal cord infarction, or chronic arterial occlusive disease; a wound such as an epithelial wound or thermal burn; sarcopenia due to aging; an arthritis such as rheumatoid arthritis, spinal disc herniation, or osteoarthritis; an inflammatory disease such as cytokine storm, nephritis, or keratitis; a mental disorder such as autism or insomnia which is expected to stem from neuroinflammation as one cause; an immunologic disease such as GVHD (graft versus host disease), Sjögren's syndrome, atopic dermatitis, connective tissue disease, multiple sclerosis, or autoimmune disease; or a cancer disease.

[G16] The therapeutic agent according to any one of [G12] to [G15], wherein the therapeutic agent is for treatment of an ischemic disease; preferably lower-limb ischemia, myocardial infarction, cerebral infarction, spinal cord infarction, or chronic arterial occlusive disease; and more preferably lower-limb ischemia.

### [EXAMPLES]

### [EXAMPLE 1] Proliferation Culture

### (1) Method

In Example 1, proliferation culture of stem cells was performed. Umbilical cord-derived mesenchymal stem cells (UCMSCs) cryopreserved in a cryopreservation solution, Stem Cell Banker (ZENOAQ) were used as stem cells.

### Experiment 1-1 (Control)

Umbilical cord-derived mesenchymal stem cells (UCMSCs) were suspended in 100 ml MSC Expansion XSFM B (FUJIFILM Wako Pure Chemical Corporation) at a cell number of 5 × 10e3 (a seeding density of 10 cells/cm²), a cell number of 5 × 10e4 (a seeding density of 100 cells/cm²), a cell number of 5 × 10e5 (a seeding density of 1000 cells/cm²), and a cell number of 5 × 10e6 (a seeding density of 10000 cells/cm²), and the obtained cell suspensions were seeded in peel-off T512 flasks (Sumitomo Bakelite Company, Limited.). The cell suspensions were seeded in four flasks under respective conditions.

During the proliferation culture, the culture medium was exchanged every 5 days. After 5 days (day 5), 10 days (day 10), 15 days (day 15), and 20 days (day 20) from the seeding, each flask was treated with TrypLE^{™} Select (Thermo Fisher Scientific Inc.) for 10 to 20 minutes to disperse the cell mass into single cells, and then the number of cells was counted with a cell counter.

FIG. 1 shows the number of proliferated cells. In FIG. 1, the vertical axis of the graph indicates a value obtained by dividing the counted number of cells by the area of the flask. FIG. 2 shows a micrograph of the cells after being cultured at a seeding density of 10 cells/cm² for 20 days.

### Experiment 1-2 (Example of Present Invention)

Umbilical cord-derived mesenchymal stem cells (UCMSCs) were suspended in 100 ml MSC Expansion XSFM B (FUJIFILM Wako Pure Chemical Corporation) at a cell number of 5 × 10e3 (a seeding density of 10 cells/cm²), a cell number of 5 × 10e4 (a seeding density of 100 cells/cm²), a cell number of 5 × 10e5 (a seeding density of 1000 cells/cm²), and a cell number of 5 × 10e6 (a seeding density of 10000 cells/cm²); iMatrix-511 laminin fragment (Nippi,Incorporated) was added in an amount of 50 µl/100 ml; and the obtained cell suspensions were seeded in peel-off T512 flasks (Sumitomo Bakelite Company, Limited). The cell suspensions were seeded in four flasks under respective conditions.

During the proliferation culture, the culture medium was exchanged every 5 days. Five days after the seeding, the iMatrix-511 laminin fragment was added again in an amount of 50 µl/100 ml. No laminin fragment was added 10 days, 15 days, and 20 days after the seeding.

After 5 days (day 5), 10 days (day 10), 15 days (day 15), and 20 days (day 20) from the seeding, each flask was treated with TrypLE^{™} Select (Thermo Fisher Scientific Inc.) for 10 to 20 minutes to disperse the cell mass into single cells, and then the number of cells was counted with a cell counter.

FIG. 3 shows the number of proliferated cells. In FIG. 3, the vertical axis of the graph indicates a value obtained by dividing the counted number of cells by the area of the flask. FIG. 4 shows a micrograph of the cells after being cultured at a seeding density of 10 cells/cm² for 20 days.

### Experiment 1-3 (Referential Example)

Umbilical cord-derived mesenchymal stem cells (UCMSCs) were suspended in 100 ml MSC Expansion XSFM B (FUJIFILM Wako Pure Chemical Corporation) at a cell number of 5 × 10e3 (a seeding density of 10 cells/cm²), a cell number of 5 × 10e4 (a seeding density of 100 cells/cm²), a cell number of 5 × 10e5 (a seeding density of 1000 cells/cm²), and a cell number of 5 × 10e6 (a seeding density of 10000 cells/cm²), and the obtained cell suspensions were seeded in peel-off T512 flasks (Sumitomo Bakelite Company, Limited) coated with gelatin in advance. Gelatin coating was performed by treating the flasks with a gelatin solution (StemSure 0.1 w/v% Gelatin Solution, FUJIFILM Wako Pure Chemical Corporation) for 2 hours.

During the proliferation culture, the culture medium was exchanged every 5 days. After 5 days (day 5), 10 days (day 10), 15 days (day 15), and 20 days (day 20) from the seeding, each flask was treated with TrypLE^{™} Select (Thermo Fisher Scientific Inc.) for 10 to 20 minutes to disperse the cell mass into single cells, and then the number of cells was counted with a cell counter.

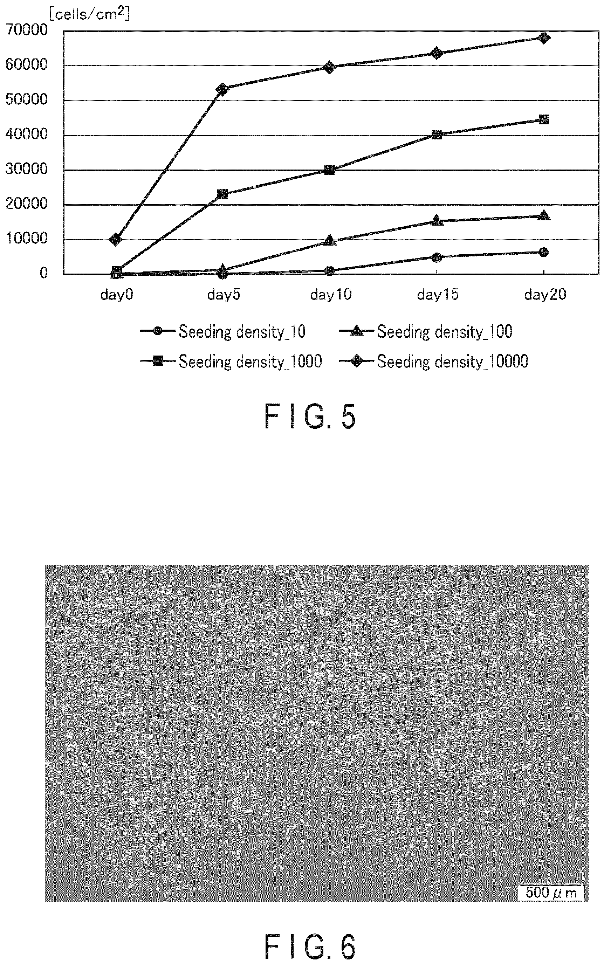

FIG. 5 shows the number of proliferated cells. In FIG. 5, the vertical axis of the graph indicates a value obtained by dividing the counted number of cells by the area of the flask. FIG. 6 shows a micrograph of the cells after being cultured at a seeding density of 10 cells/cm² for 20 days.

### (2) Results

When the umbilical cord-derived mesenchymal stem cells (UCMSCs) were proliferated to reach a confluent state in the T512 flasks, about 3 × 10e7 cells (i.e., about 60000 cells/cm²) were successfully recovered. Accordingly, FIGS. 1, 3, and 5 show that about 60000 cells/cm² are in a confluent state.

The results shown in FIG. 1 demonstrate the following: in the absence of a laminin fragment, stem cells can be proliferated to a confluent state when seeded at a seeding density of 10000 cells/cm² and cultured, but stop proliferating and cannot proliferate to a confluent state when seeded at a low cell density of 1000 cells/cm² or less and cultured. The micrograph shown in FIG. 2 demonstrates the following: in the absence of a laminin fragment, stem cells do not proliferate to a confluent state and stop proliferating in a colony state when seeded at a low cell density of 10 cells/cm² and cultured.

The results shown in FIG. 3 demonstrate the following: in the presence of a laminin fragment, stem cells can be proliferated to a confluent state even when seeded at a low cell density of 1000 cells/cm² or less and cultured. The micrograph shown in FIG. 4 also demonstrates that in the presence of a laminin fragment, stem cells can be proliferated to a confluent state even when seeded at a low cell density of 10 cells/cm² and cultured.

Furthermore, the inventors of the present application have demonstrated that umbilical cord-derived mesenchymal stem cells (UCMSCs) can also be proliferated to a confluent state when seeded at a cell density of 2 cells/cm² and cultured in a proliferation culture medium containing iMatrix-511 laminin fragment according to the same procedure as in Experiment 1-2.

The results shown in FIG. 5 demonstrate the following: in the presence of gelatin, stem cells can be proliferated to a confluent state when seeded at a seeding density of 10000 cells/cm² and cultured, but stop proliferating and cannot proliferate to a confluent state when seeded at a low cell density of 1000 cells/cm² or less and cultured. The micrograph shown in FIG. 6 demonstrates the following: in the presence of gelatin, stem cells do not proliferate to a confluent state and stop proliferating in a colony state when seeded at a low cell density of 10 cells/cm² and cultured.

These results demonstrate that when stem cells are cultured in the presence of a laminin fragment, they can be proliferated to a confluent state even when seeded at a low cell density.

### [EXAMPLE 2] Production Culture

### (1) Method

In Example 2, after performance of proliferation culture of stem cells, production culture was performed. Umbilical cord-derived mesenchymal stem cells (UCMSCs) cryopreserved in a cryopreservation solution, Stem Cell Banker (ZENOAQ) were used as stem cells.

### Experiment 2-1 (Comparative Example)

Umbilical cord-derived mesenchymal stem cells (UCMSCs) were suspended in 100 ml MSC Expansion XSFM B (FUJIFILM Wako Pure Chemical Corporation) (hereinafter also referred to as "MSC medium B") at a cell number of 5 × 10e5 (a seeding density of 1000 cells/cm²), and the obtained cell suspension was seeded in a peel-off T512 flask (Sumitomo Bakelite Company, Limited).

During the proliferation culture, the culture medium was exchanged every 5 days. After 15 days (day 15) from the seeding, the culture medium was replaced with PBS twice to wash the remaining culture medium, then subjected to the addition of a 120 ml protein-free culture medium based on DMEM/F12 culture medium and supplemented with amino acids (hereinafter also referred to as "MSC medium A"). The amino acids added were an MEM essential amino acid solution (FUJIFILM Wako Pure Chemical Corporation) and an MEM non-essential amino acid solution (FUJIFILM Wako Pure Chemical Corporation). With the addition of the MSC medium A, culturing (production culture) was performed for 3 days. After 3 days of culturing, two images of the cells were taken with an Olympus microscope. The two images were taken at different positions in the flask. The micrographs are shown in FIGS. 7A and 7B.

### Experiment 2-2 (Example of Present Invention)

Umbilical cord-derived mesenchymal stem cells (UCMSCs) were suspended in 100 ml MSC Expansion XSFM B (FUJIFILM Wako Pure Chemical Corporation) (hereinafter also referred to as "MSC medium B") at a cell number of 5 × 10e5 (a seeding density of 1000 cells/cm²); iMatrix-511 laminin fragment (Nippi, Incorporated) was added in an amount of 50 µl/100 ml; and the obtained cell suspension was seeded in a peel-off T512 flask (Sumitomo Bakelite Company, Limited).

During the proliferation culture, the culture medium was exchanged every 5 days. Five days after the seeding, the iMatrix-511 laminin fragment was added again in an amount of 50 µl/100 ml. No laminin fragment was added 10 days after the seeding.

After 15 days (day 15) from the seeding, the culture medium was replaced with PBS twice to wash the remaining culture medium, then subjected to the addition of a 120 ml protein-free culture medium based on DMEM/F12 culture medium and supplemented with amino acids (hereinafter also referred to as "MSC medium A"). The amino acids added were an MEM essential amino acid solution (FUJIFILM Wako Pure Chemical Corporation) and an MEM non-essential amino acid solution (FUJIFILM Wako Pure Chemical Corporation). With the addition of the MSC medium A, culturing (production culture) was performed for 3 days. After 3 days of culturing, two images of the cells were taken with an Olympus microscope. The two images were taken at different positions in the flask. The micrographs are shown in FIGS. 8A and 8B.

### (2) Results

FIG. 7A shows that some cells peeled off and formed a cell mass. FIG. 7A indicates the location of the cell mass with an arrow. FIG. 7B shows that most cells peeled off and did not remain. On the other hand, both of the images of FIGS. 8A and 8B show that the cells did not peel off and maintained an adhered state.

These results demonstrate that when stem cells are cultured and proliferated in the presence of a laminin fragment and the obtained proliferated cells are then cultured in a protein-free culture medium free of exogenous components, they do not peel off from the culture vessel in the middle of culturing, and the adhered state of the proliferated cells can be maintained.

### [Example 3] Long-term Production Culture

### (1) Method

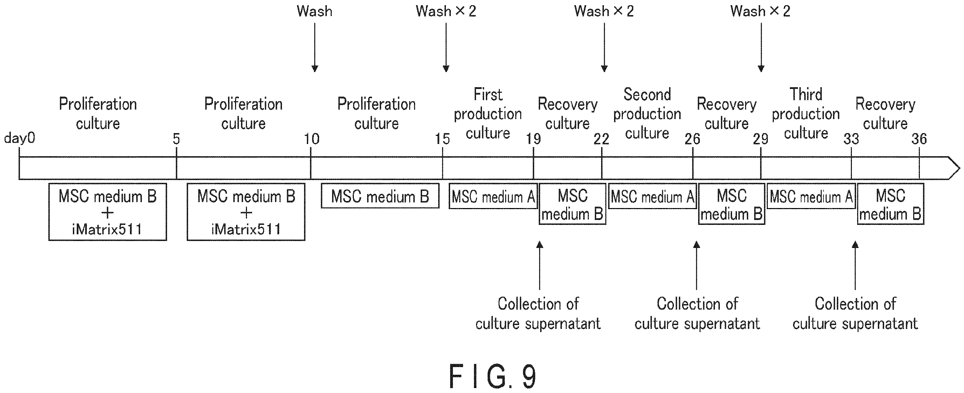

In Example 3, after the proliferation culture of stem cells was performed, long-term production culture was performed. FIG. 9 schematically shows the culture process performed in Example 3. Umbilical cord-derived mesenchymal stem cells (UCMSCs) cryopreserved in a cryopreservation solution, Stem Cell Banker (ZENOAQ) were used as stem cells.

### Experiment 3-1 (Example of Present Invention)

Umbilical cord-derived mesenchymal stem cells (UCMSCs) were suspended in 100 ml MSC Expansion XSFM B (FUJIFILM Wako Pure Chemical Corporation) (hereinafter also referred to as "MSC medium B") at a cell number of 5 × 10e5 (a seeding density of 1000 cells/cm²); iMatrix-511 laminin fragment (Nippi, Incorporated) was added in an amount of 50 µl/100 ml; and the obtained cell suspension was seeded in a peel-off T512 flask (Sumitomo Bakelite Company, Limited).

During the proliferation culture, the culture medium was exchanged every 5 days. Five days after the seeding, the iMatrix-511 laminin fragment was added again in an amount of 50 µl/100 ml. No laminin fragment was added 10 days after the seeding.

After 15 days (day 15) from the seeding, it was confirmed that the cells reached a confluent state, and the culture medium was replaced with PBS twice to wash the remaining culture medium. Thereafter, a 120 ml protein-free culture medium based on DMEM/F12 culture medium and supplemented with amino acids (hereinafter also referred to as "MSC medium A") was added. The amino acids added were an MEM essential amino acid solution (FUJIFILM Wako Pure Chemical Corporation) and an MEM non-essential amino acid solution (FUJIFILM Wako Pure Chemical Corporation). After adding the MSC medium A, culturing was performed for 4 days. This culturing is indicated as "the first production culture" in FIG. 9.

After the first production culture, a culture supernatant of the MSC medium A was collected. Cytokines contained in the culture supernatant were analyzed using an ELISA analysis kit (R&D Systems). Three types of cytokines, vascular endothelial growth factor (VEGF), interleukin-7 (IL-7), and hepatocyte growth factor (HGF), were analyzed.

After the first production culture, the cells were returned to the MSC medium B and cultured (i.e., recovery culture) for 3 days. After performing the recovery culture for 3 days, the culture medium was replaced with the MSC medium A again to perform culturing for 4 days. This culturing is indicated as "the second production culture" in FIG. 9.

After the second production culture, a culture supernatant of the MSC medium A was collected. Cytokines (VEGF, IL-7, and HGF) contained in the culture supernatant were analyzed using an ELISA analysis kit (R&D Systems).

After the second production culture, the cells were returned to the MSC medium B and cultured (i.e., recovery culture) for 3 days. After performing the recovery culture for 3 days, the culture medium was replaced with the MSC medium A again to perform culturing for 4 days. This culturing is indicated as "the third production culture" in FIG. 9.

After the third production culture, a culture supernatant of the MSC medium A was collected. Cytokines (VEGF, IL-7, and HGF) contained in the culture supernatant were analyzed using an ELISA analysis kit (R&D Systems).

As described above, the production culture was performed three times in total, and collection of the culture supernatant and analysis of the cytokines were performed three times in total.

### Experiment 3-2 (Comparative Example)

Umbilical cord-derived mesenchymal stem cells (UCMSCs) were suspended in 20 ml MSC Expansion XSFM B (FUJIFILM Wako Pure Chemical Corporation) (MSC medium B) at a cell number of 1.5 × 10e5 (a seeding density of 1000 cells/cm²), and the obtained cell suspension was seeded in a T150 flask (Corning) without the addition of iMatrix-511 laminin fragment. The proliferation culture was performed in the same procedure as in Experiment 3-1, except that no laminin fragment was added.

After 15 days (day 15) from the seeding, it was confirmed that the cells reached a confluent state, and the culture medium was replaced with the MSC medium A as in Experiment 3-1. Thereafter, the cells were cultured for 2 days, and a culture supernatant of the MSC medium A was collected. Cytokines (VEGF, IL-7, and HGF) contained in the culture supernatant were analyzed using an ELISA analysis kit (R&D Systems).

In this experiment, the culture supernatant was collected 2 days after the culture medium was replaced with the MSC medium A, since the cells would start peeling off from the culture vessel 3 days after the replacement with the MSC medium A due to no added laminin fragment.

### (2) Results

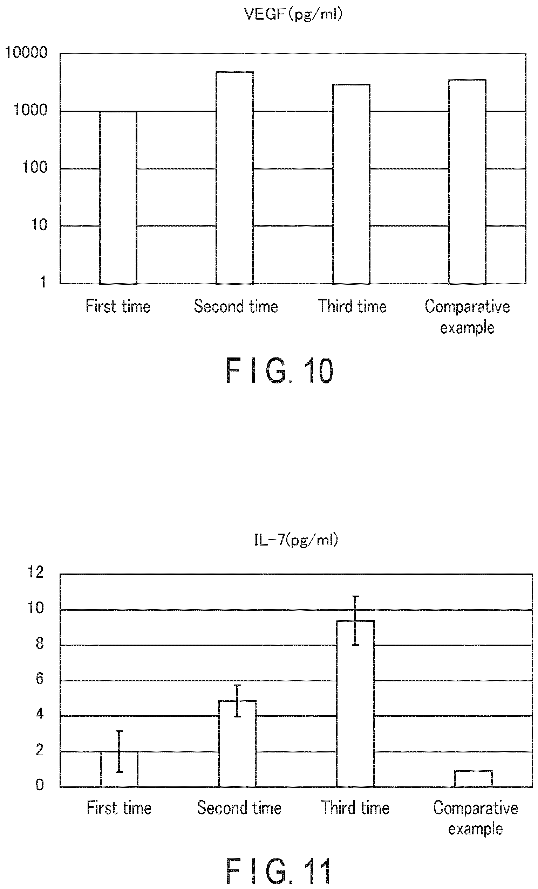

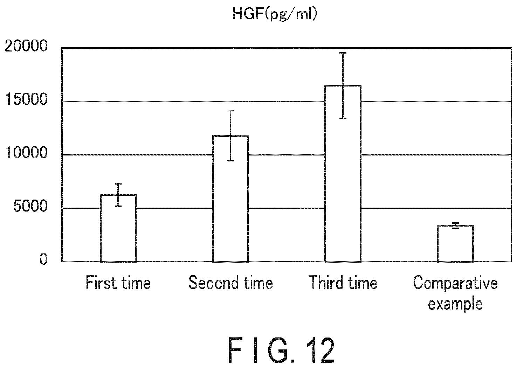

FIGS. 10 to 12 show the results of cytokine quantification. Each of the graphs of FIGS. 10 to 12 shows the following in the order from the left:
the amount of the cytokine in the culture supernatant collected after the first production culture in the example of the present invention;
the amount of the cytokine in the culture supernatant collected after the second production culture in the example of the present invention;
the amount of the cytokine in the culture supernatant collected after the third production culture in the example of the present invention; and
the amount of the cytokine in the culture supernatant collected in the comparative example.

The results shown in FIGS. 10 to 12 demonstrate that when long-term production culture is performed according to the method of the present invention, cytokines can be produced in an amount comparable to or greater than that of the comparative example, and the production amount thereof either increases or does not decrease even when the production culture is repeated. VEGF and HGF are cytokines having angiogenesis effects. Although the case was observed where the production amount of VEGF was slightly smaller than that of the comparative example, it can be said that a sufficient amount of VEGF was produced because the absolute amount of VEGF was large. The production amount of HGF tended to increase when the production culture was repeated. IL-7 is a cytokine involved in activation of immune cells and inflammation. The production amount of IL-7 tended to increase when the production culture was repeated.

In Example 3, the production culture was performed three times in total, and collection of the culture supernatant and analysis of the cytokines were performed three times in total; however, the inventors of the present application have demonstrated that cytokines can be produced by performing the production culture six times in total.

These results demonstrate the following: when stem cells are cultured and proliferated in the presence of a laminin fragment and the obtained proliferated cells are then cultured in a protein-free culture medium free of exogenous components, the production culture can be performed while maintaining the adhered state of the proliferated cells. Also, the recovery culture can be performed while maintaining the adhered state of the cells after performance of the production culture, and thus the ability of the cells to produce cell products can be recovered. Thereby, the production culture can be repeatedly performed multiple times while maintaining the adhered state of the cells, so that large amounts of cell products can be produced over a long period of time via a simple method.

### [Example 4] Mesenchymal Stem Cell Population

In Example 4, mesenchymal stem cells were cultured in the presence of a laminin fragment, and the positive rates of the cell surface markers of the obtained mesenchymal stem cell population were analyzed. In addition, mesenchymal stem cells were cultured in the presence of a laminin fragment, and the expression of the cell surface markers of the obtained mesenchymal stem cell population was ascertained with an immunostaining image.

### (1) Method

### Experiment 4-1 (Example of Present Invention)

Umbilical cord-derived mesenchymal stem cells (UCMSCs) or adipose-derived mesenchymal stem cells (ADMSCs) were suspended in 100 ml MSC Expansion XSFM B medium (FUJIFILM Wako Pure Chemical Corporation) at a cell number of 5 × 10e5 (a seeding density of 1000 cells/cm²); iMatrix-511 laminin fragment (Nippi, Incorporated) was added in an amount of 50 µl/100 ml; and the obtained cell suspension was seeded in a peel-off T512 flask (Sumitomo Bakelite Company, Limited). The umbilical cord-derived mesenchymal stem cells were cultured for 1 week, 2 weeks, and 3 weeks, and the adipose-derived mesenchymal stem cells were cultured for 1 week and 2 weeks.

After the culturing, the cells were peeled off through treatment with a TrypLE Select solution (Thermo Fisher Scientific). The obtained cells were stained with PE (phycoerythrin)-conjugated antibodies (BioLegend) of the cell surface markers CD44, CD73, CD90, CD105, and HLA-ABC, and the positive rate of each marker was analyzed using a flow cytometer (Sony).

In addition, the umbilical cord-derived mesenchymal stem cells were seeded in a 24-well plate (Corning) in the presence of a laminin fragment and cultured for one week. Thereafter, the mesenchymal stem cells adhered to the 24 wells were immunostained with PE-conjugated antibodies of the cell surface markers CD73, CD90, CD105, and HLA-ABC, and bright-field imaging and fluorescence imaging were performed with a fluorescence microscope (KEYENCE CORPORATION).

### Experiment 4-2 (Comparative Example)

Umbilical cord-derived mesenchymal stem cells (UCMSCs) were suspended in 100 ml MSC Expansion XSFM B medium (FUJIFILM Wako Pure Chemical Corporation) at a cell number of 5 × 10e5 (a seeding density of 1000 cells/cm²), and the obtained cell suspension was seeded in a peel-off T512 flask (Sumitomo Bakelite Company, Limited) without the addition of iMatrix-511 laminin fragment. The culturing was performed in the same procedure as in Experiment 4-1, except that no laminin fragment was added.

After the culturing, the positive rates of the cell surface markers CD44, CD73, CD90, CD105, and HLA-ABC were analyzed in the same procedure as in Experiment 4-1. The expression of the cell surface markers CD73, CD90, CD105, and HLA-ABC was ascertained with an immunostaining image in the same procedure as in Experiment 4-1.

### (2) Results

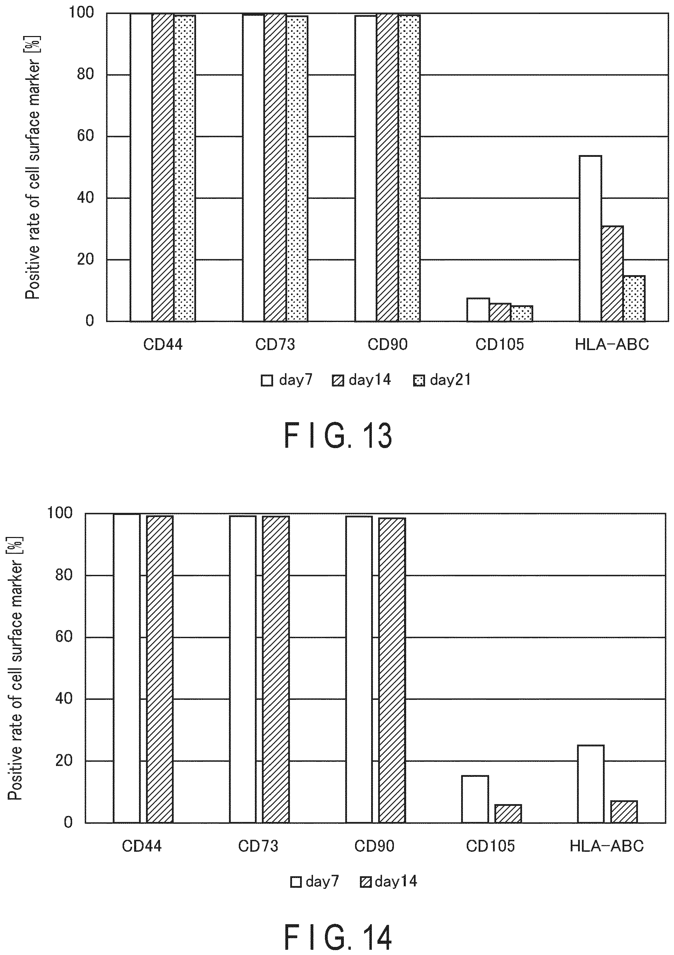

FIG. 13 shows the positive rates of the cell surface markers of the umbilical cord-derived mesenchymal stem cells cultured in the presence of a laminin fragment. FIG. 14 shows the positive rates of the cell surface markers of the adipose-derived mesenchymal stem cells cultured in the presence of a laminin fragment. FIG. 15 shows the positive rates of the cell surface markers of the umbilical cord-derived mesenchymal stem cells cultured in the absence of a laminin fragment.

When the umbilical cord-derived mesenchymal stem cells and adipose-derived mesenchymal stem cells were cultured in the presence of a laminin fragment, the positive rates of CD105 and HLA-ABC decreased significantly, but the positive rates of the markers other than CD105 and HLA-ABC did not decrease. On the other hand, when the umbilical cord-derived mesenchymal stem cells were cultured in the absence of a laminin fragment, the positive rates of CD105 and HLA-ABC did not decrease significantly.

When the umbilical cord-derived mesenchymal stem cells were cultured in the presence of a laminin fragment, CD73 and CD90 were positive in almost all of the cells, but almost no CD105 positive cells or HLA-ABC positive cells were observed, also in the results shown by the immunostaining images as in the results shown by the flow cytometer. Since the immunostaining images were taken without performing proteolytic enzyme treatment on the mesenchymal stem cells, the result demonstrates that the proteolytic enzyme treatment performed for the flow cytometer analysis did not cause a decrease in the expression of CD105 or HLA-ABC.

### [Example 5] Culture Supernatant of Mesenchymal Stem Cells

In Example 5, the proliferation culture of the mesenchymal stem cells was performed, followed by performing the production culture multiple times and collecting the culture supernatant after each production culture, according to the method described in Example 3. The amounts of cytokines, extracellular matrix, and an exosome marker contained in the culture supernatant of the obtained mesenchymal stem cells were analyzed.

### (1) Method

The amounts of cytokines and extracellular matrix contained in the culture supernatant were analyzed using an ELISA analysis kit (R&D Systems). Twenty four types of cytokines, HGF (hepatocyte growth factor), MCP-1, GRO/CXCL1, PDGF-AA, VEGF (vascular endothelial growth factor), TGF-1b, IL-4, IL-10, IL-13, IL-7, IL-15, IL-9, IL-1α, IL-1β, TNF-α, IL-8, EOTAXIN, IL-6, G-CSF, GM-CSF, MCP-3, IL-12P40, IP-10, and MIP-1α, were analyzed as the cytokines, and fibronectin was analyzed as the extracellular matrix. In addition, the amounts of exosomes contained in the culture supernatant were analyzed using a CD9/CD63 ELISA kit (Cosmo Bio Co., Ltd.) with an exosome marker protein (CD9/CD63 fusion protein) as an indicator. Herein, the amounts of cytokines, the amount of extracellular matrix, and the amount of exosome marker protein indicate values measured by the ELISA using specific antibodies.

### (2) Results

In Example 5 as well, similarly to Example 3, the proliferation culture was performed in the presence of a laminin fragment in the example of the present invention, whereas the proliferation culture was performed in the absence of a laminin fragment in the comparative example. Thus, in the example of the present invention, the production culture was repeatedly performed multiple times with success, but in the comparative example, only the first production culture was successfully performed since the cells started to peel off from the culture vessel during the first production culture.

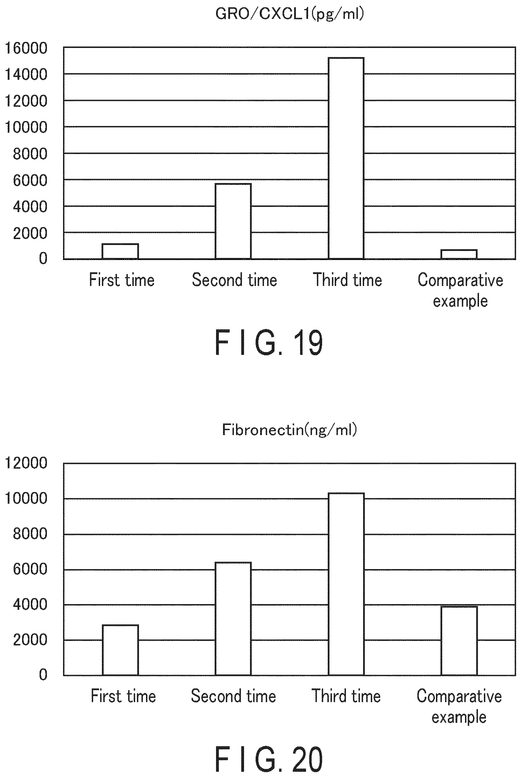

FIGS. 16 to 19 and 21 to 41 show the results of the amounts of cytokines contained in the culture supernatant, FIG. 20 shows the results of the amount of fibronectin contained in the culture supernatant, and FIG. 42 shows the results of the amount of exosome marker protein contained in the culture supernatant.

In FIGS. 16 to 42, "First Time" indicates the amounts of cytokines, fibronectin, or exosome marker protein in the culture supernatant collected after performing the first production culture (4 days) in the example of the present invention; "Second Time" indicates the amounts of cytokines, fibronectin, or exosome marker protein in the culture supernatant collected after performing the second production culture (4 days) in the example of the present invention; and "Third Time", "Fourth Time", and "Fifth Time" mean the same. Also, in FIGS. 16 to 42, "Comparative Example" indicates the amounts of cytokines, fibronectin, or exosome marker protein in the culture supernatant collected two days after the start of the first production culture in the comparative example.

The results shown in FIGS. 16 to 42 demonstrate that when the production culture was repeatedly performed and the culture supernatant was repeatedly collected according to the method of the present invention, the amounts of proteins other than IL-1α, IL-1β, and TNF-α (i.e., HGF, MCP-1, GRO/CXCL1, PDGF-AA, VEGF, TGF-1b, IL-4, IL-10, IL-13, IL-7, IL-15, IL-9, IL-8, EOTAXIN, IL-6, G-CSF, GM-CSF, MCP-3, IL-12P40, IP-10, MIP-1α, fibronectin, and exosome marker protein) tended to increase. Therefore, it is demonstrated that when the production culture is repeatedly performed and the culture supernatant is repeatedly collected according to the method of the present invention, a culture supernatant containing large amounts of cytokines, large amounts of extracellular matrix, and large amounts of exosomes can be obtained.

When a culture supernatant of mesenchymal stem cells is obtained by culturing the mesenchymal stem cells in a protein-free culture medium free of exogenous components, all the proteins contained in the culture supernatant are derived from the mesenchymal stem cells, as shown in Example 5; thus, the culture supernatant has an advantage of being easily applied as a therapeutic agent. Moreover, since the content of cytokines such as HGF, fibronectin, and exosomes in the culture supernatant of mesenchymal stem cells can be increased by repeatedly collecting the culture supernatant, the culture supernatant excels in the aspect of being expected to exhibit therapeutic effects of these proteins and exosome. The culture supernatant of mesenchymal stem cells also excels in the aspect of the content of inflammatory cytokines being relatively low because the content of IL-1α, IL-1β, and TNF-α, which are known to cause inflammation, does not increase when the culture supernatant is repeatedly collected.

### [EXAMPLE 6] Therapeutic Agent

In Example 6, the mesenchymal stem cells obtained according to the method of the present invention were administered to lower-limb ischemia rat models, and the therapeutic effects thereof were ascertained.

### (1) Method

### <Preparation of Lower-limb Ischemia Rat Models>

12-week-old Sprague Dawley (SD) male rats (purchased from CLEA Japan (Osaka, Japan)) were used. The SD rats were anesthetized by intraperitoneal administration of a combination anesthetic (M/M/B : 0.3/4/5) at a dose of 5 ml/kg. Lower-limb ischemia was induced by exposing and ligating the external iliac artery and internal iliac vein, the saphenous artery and vein at the level just below the ankle, and all branches, and excising all of the ligated blood vessels.

Three days after the induction of lower-limb ischemia, a blood flow volume was measured using laser Doppler perfusion imaging (LDPI). In order to ensure success of the induction of lower-limb ischemia and to enable evaluation of the actual effects of cell therapy by excluding rats having a significant self-regeneration ability, only the rats having a relative value (%) of 60% or less of the blood flow volume of ischemic lower limb to the blood flow volume of non-ischemic lower limb were enrolled for treatment. The enrolled rats were randomly divided into three groups: umbilical cord-derived mesenchymal stem cell (UCMSC)-administered group; adipose-derived mesenchymal stem cell (ADMSC)-administered group; and DMEM/F12-administered group.

### <Preparation and Administration of Mesenchymal Stem Cells>

Umbilical cord-derived mesenchymal stem cells (UCMSCs) were cultured in the presence and absence of a laminin fragment in the same manner as described in Example 4. Adipose-derived mesenchymal stem cells (ADMSCs) were also cultured in the presence and absence of a laminin fragment in the same manner as described in Example 4. The obtained mesenchymal stem cells (i.e., umbilical cord-derived mesenchymal stem cells cultured in the presence of a laminin fragment, umbilical cord-derived mesenchymal stem cells cultured in the absence of a laminin fragment, adipose-derived mesenchymal stem cells cultured in the presence of a laminin fragment, and adipose-derived mesenchymal stem cells cultured in the absence of a laminin fragment) were suspended in 1 ml DMEM/F12 medium (Sigma) at a cell number of 1.25 × 10e6.

The cell suspensions were administered intravenously through the tail vein with a 27-gauge needle. The administration was performed 4 times in total on the 4th day, 5th day, 6th day, and 10th day after induction of lower-limb ischemia. As a control, only DMEM/F12 medium was administered.

### <Measurement of Blood Flow Volume>

The lower limb blood flow volume was assessed using the Moor LDI 2.0 system (Moor instruments, Devon, UK). The blood flow volume was measured 14 days after the induction of lower-limb ischemia. At the time of the blood flow volume measurement, the rats were subjected to isoflurane inhalation anesthesia. The blood flow ratio (%) was calculated as a relative value using the following formula: Blood flow ratio (%) = (blood flow volume of left ischemic lower limb / blood flow volume of right non-ischemic lower limb) × 100

### (2) Results

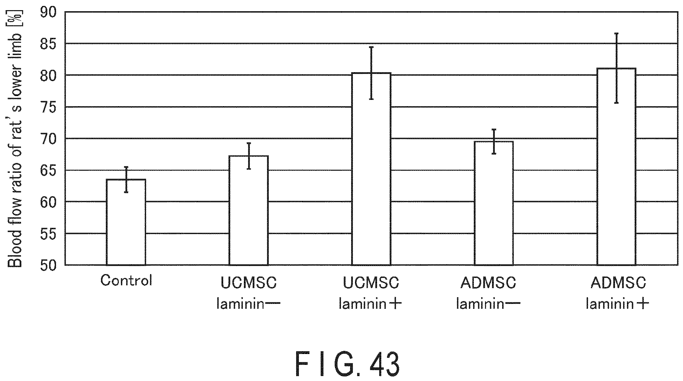

FIG. 43 shows the results of the blood flow ratio of the lower limb. In FIG. 43, "Control" represents a group administered with DMEM/F12 medium, "UCMSC laminin-" represents a group administered with umbilical cord-derived mesenchymal stem cells cultured in the absence of a laminin fragment, "UCMSC laminin+" represents a group administered with umbilical cord-derived mesenchymal stem cells cultured in the presence of a laminin fragment, "ADMSC laminin-" represents a group administered with adipose-derived mesenchymal stem cells cultured in the absence of a laminin fragment, and "ADMSC laminin+" represents a group administered with adipose-derived mesenchymal stem cells cultured in the presence of a laminin fragment. In FIG. 43, the error bars represent a standard error.

In "UCMSC laminin+" and "ADMSC laminin+", the blood flow ratio increased significantly as compared with the Control, and a significant difference from the Control was confirmed by the t-test. On the other hand, "UCMSC laminin-" and "ADMSC laminin-" showed a tendency of a slight increase in the blood flow ratio as compared with the Control, but no significant difference from the Control was confirmed by the t-test.

These results demonstrate that the mesenchymal stem cells obtained according to the method of the present invention are effective as a therapeutic agent for lower-limb ischemia. Since this therapeutic effect is deemed to be brought about by cytokines, extracellular matrices, and exosomes secreted by mesenchymal stem cells, the culture supernatant of mesenchymal stem cells obtained according to the method of the present invention is also deemed to have a similar therapeutic effect. In addition, since this therapeutic effect is deemed to be brought about by cytokines, extracellular matrices, and exosomes secreted by mesenchymal stem cells, the mesenchymal stem cells and the culture supernatant of mesenchymal stem cells obtained according to the method of the present invention are also deemed to have therapeutic effects on diseases on which cytokines are known to have therapeutic effects, diseases on which extracellular matrices are known to have therapeutic effects, and diseases on which exosomes are known to have therapeutic effects.

## Claims

1. A method of producing a cell product, the method comprising:
culturing cells in a proliferation culture medium, thereby obtaining proliferated cells in an adhered state; and
culturing the proliferated cells in a production culture medium while maintaining the adhered state, thereby causing the cells to produce a cell product.

2. The method according to claim 1, wherein the culturing cells in a proliferation culture medium is culturing cells through adhesion culture in a proliferation culture medium in a presence of a culture substrate selected from a laminin fragment having integrin-binding activity and a modified form thereof, thereby obtaining proliferated cells in an adhered state.

3. A method of producing a cell product, the method comprising:
culturing cells in a proliferation culture medium according to the method according to claim 1 or 2, thereby obtaining proliferated cells in an adhered state;
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells.

4. The method according to any one of claims 1 to 3, wherein the production culture medium is a culture medium free of a xenogeneic component.

5. The method according to any one of claims 1 to 4, wherein the production culture medium is a culture medium free of a cytokine or insulin.

6. The method according to any one of claims 1 to 5, wherein the production culture medium is a protein-free culture medium.

7. The method according to any one of claims 1 to 6, further comprising collecting a supernatant of the production culture medium after performing the culturing in the production culture medium.

8. A method of producing a culture supernatant of stem cells, the method comprising:
culturing stem cells through adhesion culture in a proliferation culture medium in a presence of a culture substrate selected from a laminin fragment having integrin-binding activity and a modified form thereof, thereby obtaining proliferated cells in an adhered state;
culturing the proliferated cells in a production culture medium while maintaining the adhered state, thereby causing the cells to produce a cell product; and
culturing the proliferated cells in a recovery culture medium while maintaining the adhered state, after performing the culturing in the production culture medium,
wherein the culturing performed in the production culture medium and the culturing performed in the recovery culture medium are alternately repeated while maintaining the adhered state of the cells, and the method further comprises collecting a supernatant of the production culture medium after performing the culturing in the production culture medium.

9. The method according to claim 8, wherein the collecting of the supernatant of the production culture medium is performed after the culturing in the production culture medium is performed for a second or subsequent time.

10. The method according to claim 8 or 9, wherein the stem cells are mesenchymal stem cells.

11. A culture supernatant of stem cells, the culture supernatant comprising 5000 pg/mL or more of HGF.

12. A culture supernatant of stem cells, the culture supernatant comprising 50 pg/mL or more of CD9/CD63 EC domain fusion protein.

13. The culture supernatant according to claim 11 or 12, further comprising 3000 pg/mL or more of MCP-1, 1000 pg/mL or more of GRO, and 5 µg/mL or more of fibronectin.

14. The culture supernatant according to any one of claims 11 to 13, further comprising 200 pg/mL or more of TGF-1b, 5 pg/mL or more of IL-4, and 8 pg/mL or more of IL-10.

15. The culture supernatant according to any one of claims 11 to 14, wherein the culture supernatant is free of at least one of insulin, transferrin, or albumin.

16. The culture supernatant according to any one of claims 11 to 15, wherein the culture supernatant is free of a recombinant protein.

17. The culture supernatant according to any one of claims 11 to 16, comprising at least one of IL-1α, IL-1β, or TNF-α in an amount of 0 to 15 pg/mL.

18. The culture supernatant according to any one of claims 11 to 17, comprising 20 or more types of cytokines, wherein each cytokine has a concentration of 10 pg/mL or more.

19. The culture supernatant according to any one of claims 11 to 18, wherein the culture supernatant is a culture supernatant of mesenchymal stem cells.

20. The culture supernatant according to any one of claims 11 to 19, wherein the culture supernatant is a culture supernatant of umbilical cord-derived mesenchymal stem cells.

21. A therapeutic agent comprising the culture supernatant according to any one of claims 11 to 20.
